# EUROPEAN PATENT APPLICATION

(11) **EP 4 797 179 A1**
(43) Date of publication of application: **26.08.2026**
(21) Application number: 24879845.6
(22) Date of filing: 18.10.2024
(51) Int. Cl.: G06Q 10/30

(54) **ESTIMATION DEVICE, ESTIMATION METHOD, ESTIMATION SYSTEM, EVALUATION METHOD, AND COMPUTER PROGRAM**

(30) Priority: 20.10.2023 JP 2023181202
(71) Applicant: Nippon Steel Corporation, Tokyo 100-8071 (JP)
(72) Inventor: HIRANO Koji, Tokyo 100-8071 (JP); TATEMIZO Nobuyuki, Tokyo 100-8071 (JP)
(74) Representative: Zimmermann & Partner Patentanwälte mbB
(86) International application number: PCT/JP2024/037268
(87) International publication number: WO 2025/084425

(57) **Abstract**

A prediction device includes a control unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap.

## Description

### TECHNICAL FIELD

The present invention relates to a prediction device, a prediction method, a prediction system, a determination method, and a computer program. Priority is claimed on Japanese Patent Application No. 2023-181202, filed on October 20, 2023, the content of which is incorporated herein by reference.

### BACKGROUND ART

In recent years, in the steel industry, there has been a growing demand to recycle iron with the aim of achieving effects such as reducing carbon dioxide emissions. There are a plurality of types of scrap containing iron (ferrous scrap) as a recycling target. New scrap generated mainly in the working process of steel products is classified as high-grade scrap, whereas materials such as H2, produced primarily from building demolition, are classified as low-grade scrap. In order to produce high-grade steel, it is desirable to use the high-grade scrap, but relying solely on the high-grade scrap poses a risk of a future supply shortage. Therefore, it is necessary to utilize the low-grade scrap.

The low-grade scrap contains a high proportion of impurity elements other than iron, and the variation in their content is large. This is because the influence of the contamination with prohibited objects such as motors and power switchboards mixed into the scrap is large. Among the impurity elements, in a case where element components (tramp element (TE), representative examples: Cu, Sn, Ni, Cr, and Mo) that are difficult to remove in a steelmaking process are mixed into the steel, the element components can cause cracking during hot rolling and lead to nonconformity of material (mechanical properties). In order to solve such an issue, a technology of measuring a concentration of a specific impurity element after dissolution has been proposed (for example, see Patent Documents 1 and 2).

### Citation List

### Patent Document

Patent Document 1: Japanese Unexamined Patent Application, First Publication No. 2007-322190
Patent Document 2: Japanese Unexamined Patent Application, First Publication No. 2011-168823

### SUMMARY OF INVENTION

### Technical Problem

However, in the technology of measuring the concentration after dissolution in the related art, it is difficult to remove the TE even in a case where the TE is incorporated at levels above a standard.

Therefore, the present invention has been made in view of the above circumstances, and provides a technology capable of acquiring the TE component in the iron that is the recycling target with higher accuracy.

### Solution to Problem

(1) One aspect of the present invention relates to a prediction device including: a control unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap.
(2) One aspect of the present invention relates to the prediction device according to (1), in which the supplementary information includes attribute information of the ferrous scrap itself or information related to an inspection performed in advance on the ferrous scrap.
(3) One aspect of the present invention relates to the prediction device according to (1) or (2), in which the supplementary information includes information related to an external appearance of the ferrous scrap.
(4) One aspect of the present invention relates to the prediction device according to (1), in which the supplementary information includes information related to prohibited objects contained in the ferrous scrap.
(5) One aspect of the present invention relates to the prediction device according to (4), in which the information related to the prohibited objects contained in the ferrous scrap indicates number or types of the prohibited objects predicted by using a prohibited-object prediction model that is built in advance and is used to predict the prohibited objects.
(6) One aspect of the present invention relates to a prediction method including: a prediction step of predicting a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap.
(7) One aspect of the present invention relates to a computer program for causing a computer to function as a prediction device including: a control unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap.
(8) One aspect of the present invention relates to a prediction system including: a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and a removal target determination unit configured to determine a prohibited object to be removed from the ferrous scrap based on a prediction result obtained by the prediction unit.
(9) One aspect of the present invention relates to the prediction system according to (8), in which the supplementary information includes information related to an external appearance of the ferrous scrap.
(10) One aspect of the present invention relates to a determination method including: a prediction step of predicting a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and a removal target determination step of determining a prohibited object to be removed from the ferrous scrap based on a prediction result obtained in the prediction step.
(11) One aspect of the present invention relates to a computer program for causing a computer to function as a prediction system including: a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and a removal target determination unit configured to determine a prohibited object to be removed from the ferrous scrap based on a prediction result obtained by the prediction unit.
(12) One aspect of the present invention relates to a prediction system including: a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and a blend information determination unit configured to determine a ferrous scrap blend such that a predetermined condition is satisfied, based on the prediction result obtained by the prediction unit and a price of the ferrous scrap.
(13) One aspect of the present invention relates to a determination method including: a prediction step of predicting a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and a blend information determination step of determining a ferrous scrap blend such that a predetermined condition related to the ferrous scrap is satisfied, based on the prediction result obtained in the prediction step and a price of the ferrous scrap.
(14) One aspect of the present invention relates to a computer program for causing a computer to function as a prediction system including: a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and a blend information determination unit configured to determine a ferrous scrap blend such that a predetermined condition is satisfied, based on the prediction result obtained by the prediction unit and a price of the ferrous scrap.
(15) One aspect of the present invention relates to a prediction system including: a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and a price determination unit configured to determine a price of the ferrous scrap in accordance with a prediction result obtained by the prediction unit.
(16) One aspect of the present invention relates to a determination method including: a prediction step of predicting a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and a price determination step of determining a price of the ferrous scrap in accordance with a prediction result obtained in the prediction step.
(17) One aspect of the present invention relates to a computer program for causing a computer to function as a prediction system including: a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and a price determination unit configured to determine a price of the ferrous scrap in accordance with a prediction result obtained by the prediction unit.

### Advantageous Effects of Invention

According to the present invention, it is possible to acquire the concentration of the impurity elements in the iron that is the recycling target with higher accuracy.

### BRIEF DESCRIPTION OF DRAWINGS

[FIG. 1] A schematic block diagram showing a system configuration of a prediction system 100 according to a first embodiment of the present invention.
[FIG. 2] A diagram showing an application example of the prediction system 100 according to the present embodiment.
[FIG. 3] A schematic block diagram showing a specific example of a functional configuration of a terminal device 10.
[FIG. 4] A schematic block diagram showing a specific example of a functional configuration of a prediction device 30.
[FIG. 5] A diagram showing a first specific example of training data.
[FIG. 6] A diagram showing an example of a prediction model obtained through training using the training data as shown in FIG. 5.
[FIG. 7] A diagram showing a second specific example of the training data.
[FIG. 8] A diagram showing an example of a prediction model obtained through training using the training data as shown in FIG. 7.
[FIG. 9] A diagram showing a third specific example of the training data.
[FIG. 10] A diagram showing an example of a prediction model obtained through training using the training data as shown in FIG. 9.
[FIG. 11] A diagram showing a fourth specific example of the training data.
[FIG. 12] A diagram showing an example of a prediction model obtained through training using the training data as shown in FIG. 11.
[FIG. 13] A flowchart showing a specific example of processing of the prediction device 30.
[FIG. 14] A schematic block diagram showing a specific example of a functional configuration of a model building device 40.
[FIG. 15] A flowchart showing a specific example of processing of the model building device 40.
[FIG. 16] A diagram showing a modification example of the prediction device 30.
[FIG. 17] A diagram showing a modification example of the prediction device 30.
[FIG. 18] A schematic block diagram showing a system configuration of the prediction system 100 according to a second embodiment of the present invention.
[FIG. 19] A schematic block diagram showing a specific example of a functional configuration of a prohibited-object prediction device 50.
[FIG. 20] A flowchart showing a specific example of processing of the prohibited-object prediction device 50.
[FIG. 21] A diagram showing a modification example of the prediction device 30.
[FIG. 22] A schematic block diagram showing a system configuration of the prediction system 100 according to a third embodiment of the present invention.
[FIG. 23] A schematic block diagram showing a specific example of a functional configuration of a removal target determination device 60.
[FIG. 24] A diagram showing a specific example of prohibited-object information.
[FIG. 25] A flowchart showing a specific example of processing of a removal target determination unit 632.
[FIG. 26] A diagram showing an application example of the prediction system 100 according to the present embodiment.
[FIG. 27] A schematic block diagram showing a system configuration of the prediction system 100 according to a fourth embodiment of the present invention.
[FIG. 28] A schematic block diagram showing a specific example of a functional configuration of a blend information determination device 70.
[FIG. 29] A diagram showing a specific example of inventory information.
[FIG. 30] A schematic block diagram showing a system configuration of the prediction system 100 according to a fifth embodiment of the present invention.
[FIG. 31] A schematic block diagram showing a specific example of a functional configuration of a price determination device 80.
[FIG. 32] A diagram showing a specific example of price information.
[FIG. 33] A diagram showing a schematic example of a hardware configuration of an information processing device 90 applied to the present embodiment.

### DESCRIPTION OF EMBODIMENTS

### [First Embodiment]

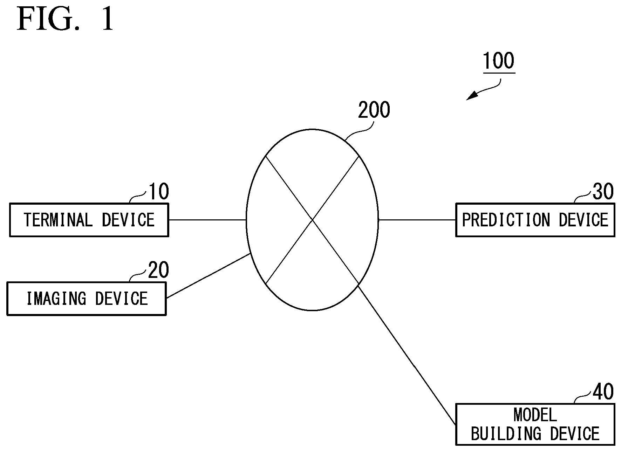

Specific configuration examples of the present invention will be described with reference to the accompanying drawings. FIG. 1 is a schematic block diagram showing a system configuration of a prediction system 100 according to a first embodiment of the present invention. The prediction system 100 is used when predicting a concentration of impurity elements contained in ferrous scrap that is a prediction target (hereinafter, referred to as "target scrap"). In the following description, copper will be described as a specific example of the impurities contained in the target scrap. However, the types of the impurities that can be predicted by using the prediction system 100 are not limited to copper. In the following description, the impurities indicate elements (for example, copper) other than an iron element, and a prohibited object indicates a part (for example, a motor) other than iron in the scrap.

The prediction system 100 includes a terminal device 10, an imaging device 20, a prediction device 30, and a model building device 40. The terminal device 10, the imaging device 20, and the prediction device 30 are communicably connected to each other via a network 200. The prediction device 30 and the model building device 40 may be communicably connected to each other via the network 200. The network 200 may be a network that uses wireless communication or a network that uses wired communication. The network 200 may be configured using, for example, the Internet or a local area network (LAN). The network 200 may be configured by combining a plurality of networks. The network used when the terminal device 10 and the prediction device 30 communicate with each other and the network used when the imaging device 20 and the prediction device 30 communicate with each other may be different networks.

First, an overall view of a scrap distribution channel to which the prediction system 100 according to the present embodiment is applied will be described. First, ferrous scrap is generated by demolishing or discarding a structure made of iron. The structure may be demolished by, for example, a demolition contractor. In this case, information related to the structure before the demolition or the discarding, information related to the demolition contractor, information indicating a situation during the demolition, information indicating a situation during the discarding, and the like may be handled as a part of supplementary information.

The generated ferrous scrap is collected by a collection operator. The collected ferrous scrap is processed by a scrap processing operator. These collection operator and scrap processing operator are collectively referred to as scrap dealers. The scrap dealer may capture an image of the ferrous scrap during the collection (reception). In this case, the captured image may be handled as a part of the supplementary information. The scrap dealer sorts the received ferrous scrap and transports the sorted ferrous scrap to a scrap user. In a case where the received ferrous scrap is large, the scrap dealer may process the ferrous scrap. Such processing includes, for example, shearing (guillotine or gas torch), shredding (fragmentation), and press working. The ferrous scrap has a smaller shape by being processed.

In the sorting of the ferrous scrap, an inferred value of tramp element components contained in each ferrous scrap by the prediction system 100 may be used. By performing the sorting using such an inferred value, the scrap dealer can predict a concentration of the tramp element components contained in the ferrous scrap in advance, and set and achieve a sorting target that satisfies a request of a purchaser (scrap user). Information related to the scrap dealer that has handled the ferrous scrap, information related to the processing on the ferrous scrap, information related to a contact person of the scrap dealer, information indicating a result of the sorting of the ferrous scrap, and the like may be handled as a part of the supplementary information.

The ferrous scrap user that receives the transport of the scrap receives the transport of the ferrous scrap from the scrap dealer and purchases some or all of the ferrous scrap. In this case, an image of the ferrous scrap captured during the transport, information related to a person who has transported the ferrous scrap, and the like may be handled as a part of the supplementary information. A purchase price may be determined by predetermined criteria. For example, the purchase price may be determined based on a shape or a size of the ferrous scrap, or may be determined in accordance with the tramp element components contained in the ferrous scrap. The tramp element components may be predicted by the prediction system 100 using the supplementary information described above. The purchased ferrous scrap is managed for each lot, a blend ratio is determined after the ferrous scrap is finely crushed, and the ferrous scrap is dissolved by an electric furnace. In this case, the purchase price, an image captured during the crushing, and the like may be used as a part of the supplementary information. The scrap user can obtain a desired steel component by predicting the tramp element components contained in the purchased ferrous scrap for each lot by the prediction system 100.

An actual measurement value of the tramp element components may be obtained by performing component analysis on the dissolved ferrous scrap. The actual measurement value may be fed back to the prediction system 100, and a prediction model may be updated using the prediction result and the actual measurement value.

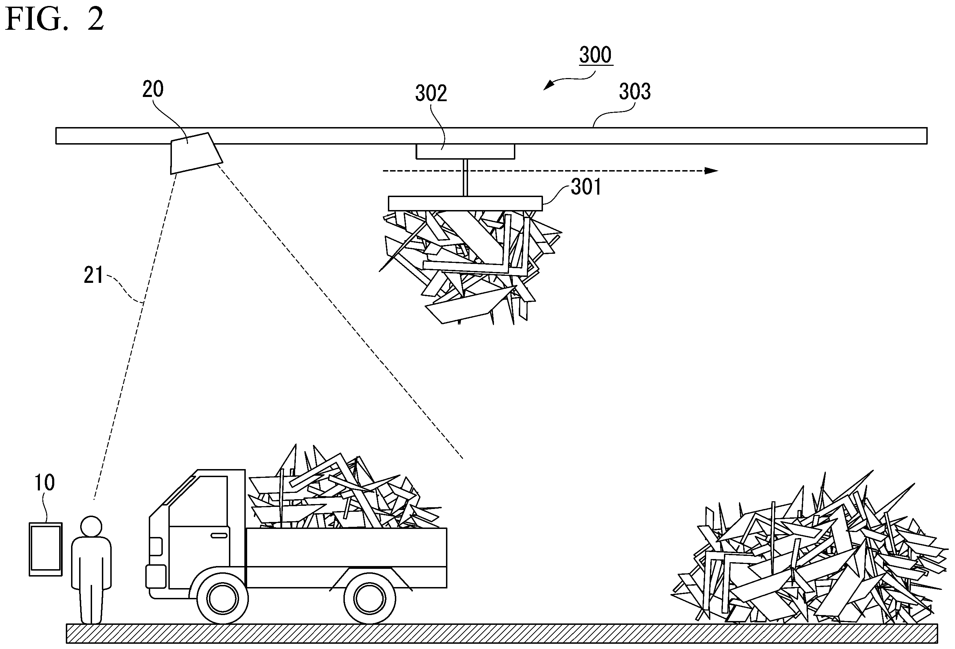

FIG. 2 is a diagram showing an application example of the prediction system 100 according to the present embodiment (in which the terminal device 10 and the imaging device 20 are not shown). In the application example shown in FIG. 2, the prediction system 100 is applied to a ferrous scrap yard that is a place where the ferrous scrap (target scrap) that is a recycling target is accumulated. The ferrous scrap yard is provided with the imaging device 20 and a transport device 300. The imaging device 20 is provided at, for example, a position where the ferrous scrap loaded on a bed of a truck that transports the ferrous scrap can be captured in a site of the demolition contractor, the scrap dealer, the ferrous scrap user, and the like described above. For example, the imaging device 20 may be provided to capture the bed of the truck parked at a predetermined parking position. The transport device 300 is provided to transport the ferrous scrap from the bed of the truck to a ferrous scrap loading area. The transport device 300 includes, for example, a lifting magnet 301, a crane 302, and a crane rail 303.

The ferrous scrap generated by demolishing a building at factories or within cities is transported to the ferrous scrap yard by the truck. The truck in which the ferrous scrap is loaded on the bed is parked at the predetermined parking position. When the truck is parked, the imaging device 20 captures an image of a region including the bed to generate image data. The imaging device 20 transmits the generated image data to the prediction device 30. In a case where the imaging by the imaging device 20 is completed, the transport device 300 transports the ferrous scrap from the bed of the truck to the ferrous scrap loading area.

In the ferrous scrap yard, a worker who performs work related to loading and unloading of the ferrous scrap operates the terminal device 10 to input inspection record information as the supplementary information. The inspection record information includes information (for example, a profile) related to a delivery contractor, information indicating where the ferrous scrap is generated, and the like. The delivery contractor may operate the terminal device 10 to input the inspection record information. The terminal device 10 receives the input of the inspection record information in accordance with the operation of the worker, and transmits the input inspection record information to the prediction device 30 (not shown). The prediction device 30 performs prediction processing using the supplementary information (including the inspection record information and/or the image data) acquired by the demolition contractor, the scrap dealer, the ferrous scrap user, and the like described above. The prediction device 30 transmits the prediction result obtained by the prediction processing to the terminal device 10. In a case where the terminal device 10 receives the prediction result from the prediction device 30, the terminal device 10 displays the received prediction result. Next, each device used in the prediction system 100 will be described.

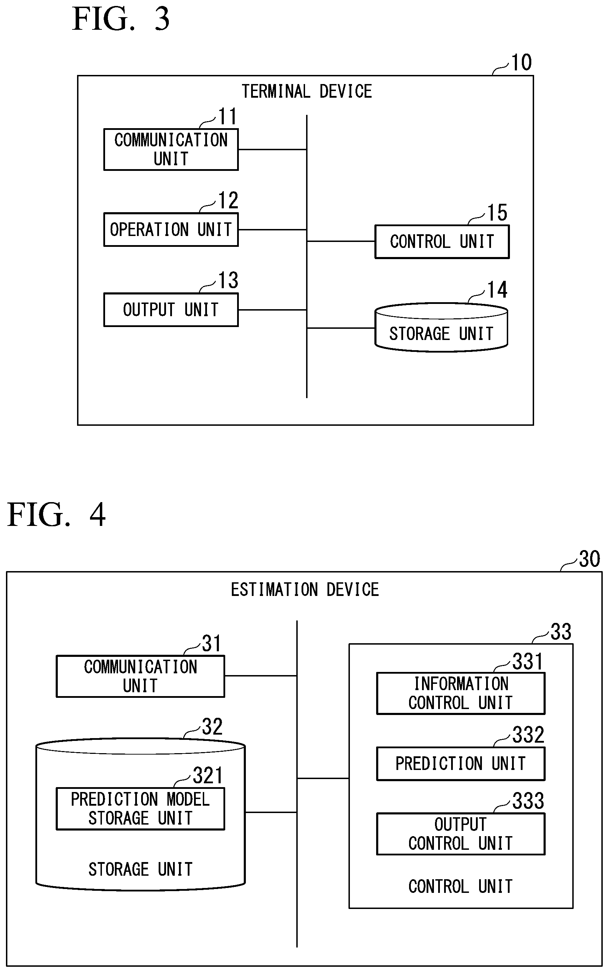

FIG. 3 is a schematic block diagram showing a specific example of a functional configuration of the terminal device 10. The terminal device 10 is configured using, for example, an information device such as a smartphone, a tablet, a personal computer, or a dedicated device. The terminal device 10 includes a communication unit 11, an operation unit 12, an output unit 13, a storage unit 14, and a control unit 15.

The communication unit 11 is a communication device. The communication unit 11 may be, for example, configured as a network interface. The communication unit 11 performs data communication with another device via the network 200 under the control of the control unit 15. The communication unit 11 may be a device that performs wireless communication or a device that performs wired communication.

The operation unit 12 is configured using an existing input device such as a keyboard, a pointing device (a mouse, a tablet, and the like), a button, and a touch panel. The operation unit 12 is operated by a user when the user inputs an instruction to the terminal device 10. The operation unit 12 may be an interface for connecting the input device to the terminal device 10. In this case, the operation unit 12 inputs an input signal generated in accordance with the input of the user using the input device to the terminal device 10. The operation unit 12 may be configured using a microphone and a voice recognition device. In this case, the operation unit 12 performs voice recognition on a sentence uttered by the user and inputs character string information of the recognition result to the terminal device 10. In this case, the operation unit 12 may perform only the input of the voice, and the voice recognition may be executed by the control unit 15. The operation unit 12 may be configured in any manner as long as the operation unit 12 can input the instruction of the user to the terminal device 10.

The output unit 13 outputs the information in a form that can be recognized by the user. The output unit 13 may be, for example, an image display device such as a liquid-crystal display or an organic electro luminescence (EL) display. The output unit 13 may be an interface for connecting the image display device to the terminal device 10. In this case, the output unit 13 generates a video signal for displaying the image data, and outputs the video signal to an image display device connected to the output unit 13. The output unit 13 may be a device that outputs sound, such as a speaker. The output unit 13 may be an interface for connecting an acoustic output device such as a speaker or headphones to the terminal device 10. In this case, the output unit 13 generates an acoustic signal for reproducing acoustic data, and outputs the acoustic signal to the acoustic output device connected to the output unit 13.

The storage unit 14 is configured using a storage device such as a magnetic hard disk device or a semiconductor storage device. The storage unit 14 stores data used by the control unit 15. The storage unit 14 stores data required for the control unit 15 to perform processing.

The control unit 15 is configured using one or more hardware processors such as a central processing unit (CPU) and one or more memories (main storage devices). The memory is configured using, for example, a storage device such as a random access memory (RAM) or a read only memory (ROM). The control unit 15 functions by causing the one or more hardware processors to perform various computations through execution of one or more programs stored in the memories. All or some of the functions of the control unit 15 may be implemented by using hardware such as an application specific integrated circuit (ASIC), a programmable logic device (PLD), or a field programmable gate array (FPGA). The above-described program may be recorded on a computer-readable recording medium. The computer-readable recording medium is, for example, a portable medium such as a flexible disk, a magneto-optical disk, a ROM, a CD-ROM, or a semiconductor storage device (for example, a solid state drive (SSD)), or a storage device such as a hard disk or a semiconductor storage device built in a computer system. The above-described program may be transmitted via a telecommunications line.

The control unit 15 may execute, for example, an application installed in the device (terminal device 10) itself. As a specific example of such an application, there is an application provided to the terminal device 10 as a dedicated application for the prediction system 100. As another specific example of such an application, there is an application of a web browser. Such an application may be installed in the terminal device 10 in advance, or may be downloaded each time the prediction processing is executed. For example, in a case where the application is implemented as the application of the web browser, the application may be configured as follows. The terminal device 10 downloads the application from a device designated by the web server in accordance with the connection of the terminal device 10 to a specific web server. Then, the terminal device 10 executes the downloaded application. The device designated by the web server may be, for example, the web server itself or another server. The control unit 15 operates in accordance with a program of the application being executed.

The control unit 15 controls the terminal device 10 in accordance with the operation of the user or the information received from the prediction device 30. For example, the control unit 15 transmits the information input by the user operating the operation unit 12 to the prediction device 30 by using the communication unit 11. For example, in a case where the information transmitted from the prediction device 30 is received by the communication unit 11 via the network 200, the control unit 15 generates screen data based on the received information and displays the screen data on the output unit 13. The screen data includes an image or characters transmitted from the prediction device 30. For example, in a case where the information transmitted from the prediction device 30 is received by the communication unit 11 via the network 200, the control unit 15 generates audio data based on the received information and outputs the audio data from the output unit 13.

Hereinafter, a specific example of the operation of the control unit 15 will be described. In the following example, the image display device is used as a specific example of the output unit 13. However, as described above, the output unit 13 does not need to be configured using the image display device, and may be configured using a voice output device or may be configured using both the image display device and the voice output device.

The control unit 15 generates screen data having characters or an image that instructs the user to input the information required for the prediction device 30 to execute the prediction processing. The control unit 15 displays the generated screen data on the output unit 13. As the information for which the user is instructed to input, for example, supplementary information related to the target scrap is provided. The user operates the operation unit 12 to input the supplementary information for which the user is instructed to input, to the terminal device 10. For example, in a case where the supplementary information is input, the control unit 15 may instruct the user to input a command to start the prediction processing. The user operates the operation unit 12 to input an instruction to transmit the command to start the processing to the prediction device 30 to the terminal device 10. The control unit 15 transmits the input supplementary information and the instruction to start the prediction processing to the prediction device 30 by using the communication unit 11.

The control unit 15 receives the prediction result from the prediction device 30. The control unit 15 generates screen data indicating information on the prediction result. The control unit 15 displays the generated screen data on the output unit 13. By checking such display, the user can easily know information related to an amount of the impurities (copper) contained in the ferrous scrap (target scrap) that is the prediction target.

The imaging device 20 acquires information (external appearance information) related to an external appearance of the target scrap by imaging the target scrap. The imaging device 20 may be configured using, for example, a camera (image sensor) that receives visible light obtained from a space including the target scrap to generate two-dimensional image data. Such a camera is a camera that performs so-called imaging on the target scrap. The imaging device 20 may be a sensor that acquires information related to a three-dimensional shape of the target scrap. In this case, the imaging device 20 may be configured using a device that measures a distance from the imaging device 20 itself to each point on the surface of the target scrap. More specifically, the imaging device 20 may be configured using a measurement device that measures a distance from the imaging device 20 to each portion of the surface of the target scrap by irradiating the target scrap with laser and measuring scattered light. As a specific example of such an imaging device 20, there is laser imaging detection and ranging (LIDAR). The imaging device 20 transmits the acquired external appearance information to the prediction device 30.

The prediction device 30 predicts the concentration of the impurity elements contained in the target scrap. The prediction device 30 is configured using, for example, an information processing device such as a personal computer or a server device. FIG. 4 is a schematic block diagram showing a specific example of a functional configuration of the prediction device 30. As in the shown example, the prediction device 30 includes a communication unit 31, a storage unit 32, and a control unit 33.

The communication unit 31 is a communication device. The communication unit 31 may be, for example, configured as a network interface. The communication unit 31 performs data communication with another device via the network 200 under the control of the control unit 33. The communication unit 31 may be a device that performs wireless communication or a device that performs wired communication.

The storage unit 32 is configured using a storage device such as a magnetic hard disk device or a semiconductor storage device. The storage unit 32 stores data used by the control unit 33. The storage unit 32 may function as, for example, a prediction model storage unit 321.

The prediction model storage unit 321 stores information (hereinafter, referred to as an "prediction model") used to predict the concentration of the impurity elements contained in the target scrap. The prediction model is generated in advance by model building processing. Such model building processing may be executed, for example, by another device (for example, the model building device 40), by the device (prediction device 30) itself, or by human labor. The prediction model stored in the prediction model storage unit 321 will be described below.

The prediction model may be obtained by, for example, the model building processing using known data. The prediction model may be information indicating a trained model obtained by performing supervised learning processing using a plurality of known data (training data) having ground truth labels, information obtained by performing statistical processing using known data, or other types of information. Specific examples of the learning processing include multivariate analysis, so-called machine learning, and deep learning. As the learning processing, learning processing for classification may be used or learning processing for regression may be used in accordance with the ground truth label used in the training data. The control unit 33 predicts the concentration of the impurity elements contained in the target scrap by using the prediction model stored in the prediction model storage unit 321. The training data in the present embodiment will be described below.

As the training data, a combination of the supplementary information and the ground truth label (information indicating the concentration of the impurity elements) is used for each aggregate (hereinafter, referred to as a "lot") in which the ferrous scrap of a predetermined unit is set as one aggregate. In this case, the supplementary information corresponds to an explanatory variable, and the information indicating the concentration of the impurity elements corresponds to a response variable. The lot may be, for example, the following aggregate. An aggregate of the ferrous scrap that is loaded on the same truck when the ferrous scrap is brought to the loading/unloading area. An aggregate of the ferrous scrap that is brought in from the same scrap dealer within a predetermined period (for example, 15 minutes, 30 minutes, or 1 hour). An aggregate of the ferrous scrap that is loaded on a plurality of trucks designated by the scrap dealer. An aggregate of the scrap that adheres to a lifting magnet in a single application of magnetic force during the unloading process. The lot may be defined in any manner, provided it is an aggregate of the ferrous scrap predicted to contain a comparable amount of impurities; for example, the lot may be an aggregate corresponding to an amount of the ferrous scrap that can be transported in a single operation by a transport device such as a crane or other heavy equipment. In the following description, the aggregate of the ferrous scrap that is loaded on the same truck when the ferrous scrap is transported to the loading/unloading area will be described as one lot.

The supplementary information is information related to the ferrous scrap. The supplementary information includes, for example, the inspection record information or the external appearance information. The inspection record information includes attribute information of the ferrous scrap itself or information (including an inspection condition and an inspection result) related to the inspection performed in advance on the ferrous scrap. The data format of the inspection record information may be represented in any form such as a character string, a numerical value, and an image. The attribute information is, for example, information indicating a generation source (demolished building) of the ferrous scrap or information indicating a generation time (time when the demolition is performed) of the ferrous scrap. The information related to the inspection is information indicating the delivery contractor of the ferrous scrap, the demolition contractor of the ferrous scrap, a person in charge (hereinafter, referred to as a "sorting operator") who has performed work (sorting work) of removing foreign substances from the ferrous scrap, a delivery date and time, a grade of the ferrous scrap, and the like.

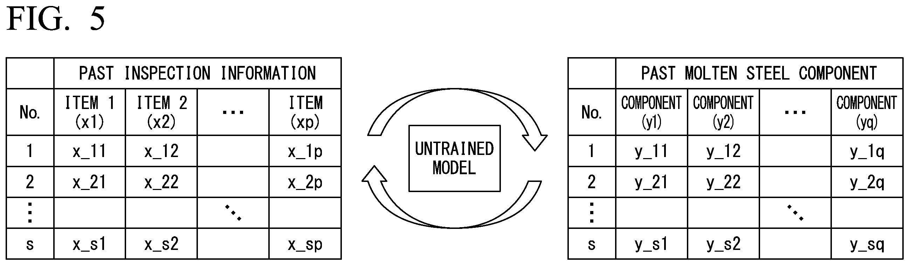

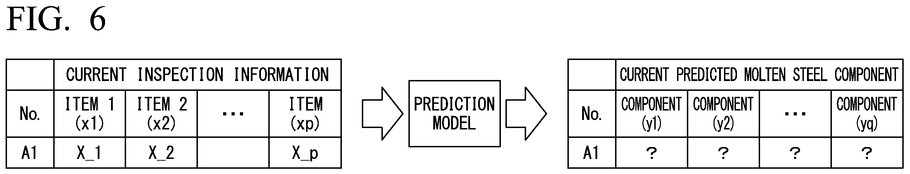

FIG. 5 is a diagram showing a first specific example of the training data. The training data shown in FIG. 5 is an example in which past inspection information is used as an explanatory variable and past molten steel component is used as a response variable. The past inspection information includes values of a plurality of inspection items (x1 to xp) for each lot indicated as No. The past molten steel component has values of components (y1 to yq) actually molten and measured for each lot of the past inspection information. The prediction model may be generated by training an untrained model using these values. FIG. 6 is a diagram showing an example of the prediction model obtained by being trained using the training data as shown in FIG. 5. A current predicted molten steel component, which is a response variable, is obtained by inputting current inspection information, which is an explanatory variable, to the prediction model. The current inspection information indicates values of the items x1 to xp related to the ferrous scrap that is the prediction target. The current predicted molten steel component indicates a predicted value of each component related to the ferrous scrap that is the prediction target.

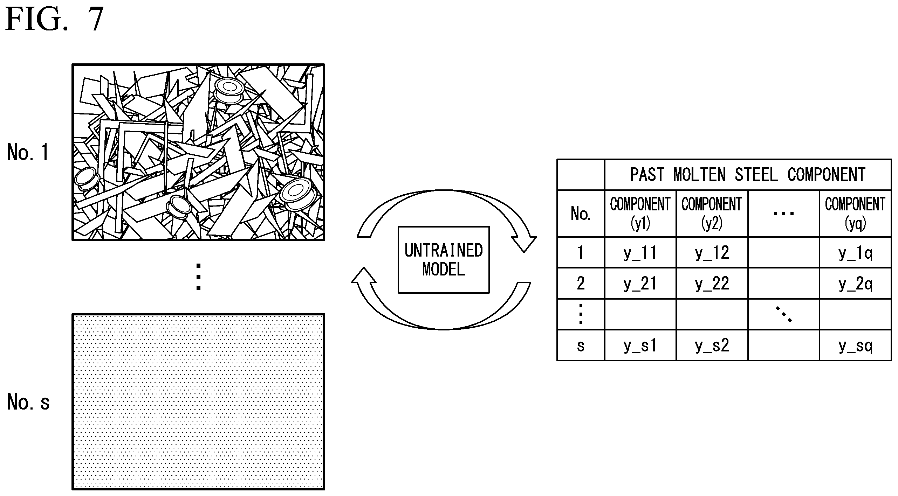

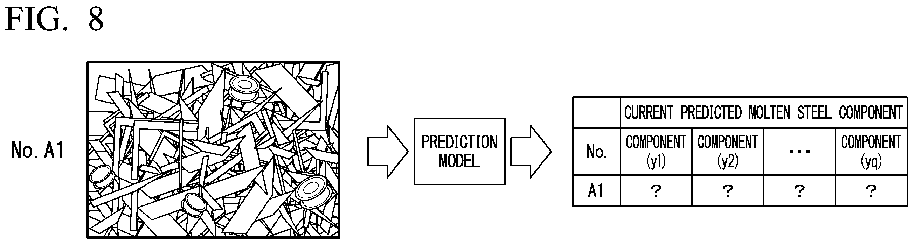

FIG. 7 is a diagram showing a second specific example of the training data. The training data shown in FIG. 7 is an example in which a past image is used as an explanatory variable and a past molten steel component is used as a response variable. The past image is one or a plurality of images captured in a predetermined step for each lot indicated as No. In the example of FIG. 7, one image is used for each lot. The past molten steel component includes values of components (y1 to yq) actually molten and measured for each lot of the past image. The prediction model may be generated by training an untrained model using these values. FIG. 8 is a diagram showing an example of the prediction model obtained by being trained using the training data as shown in FIG. 7. A current predicted molten steel component, which is a response variable, is obtained by inputting a current image (No. A1), which is an explanatory variable, to the prediction model. The current image indicates an image captured in a predetermined step for the ferrous scrap that is the prediction target. The current predicted molten steel component indicates a predicted value of each component related to the ferrous scrap that is the prediction target.

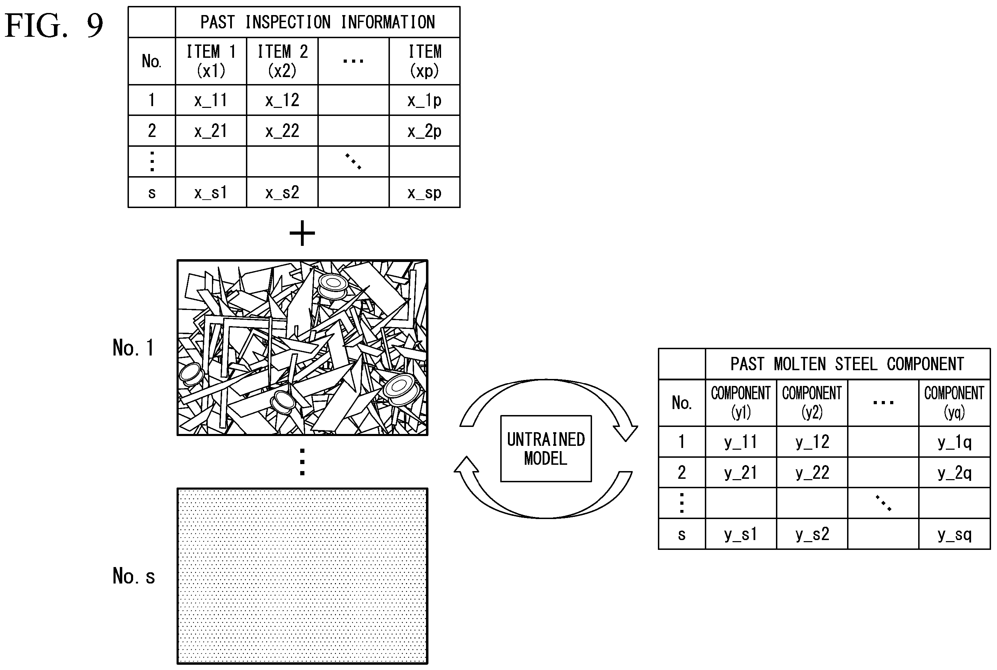

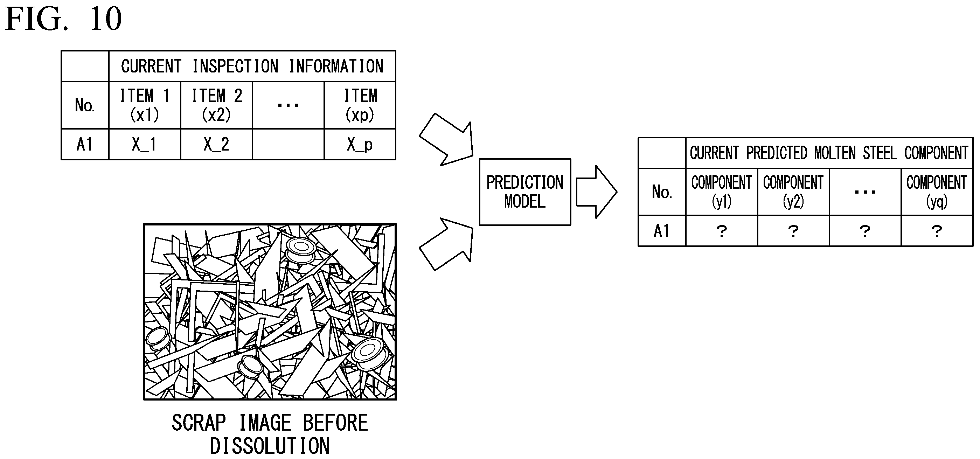

FIG. 9 is a diagram showing a third specific example of the training data. The training data shown in FIG. 9 is an example in which the past inspection information and the past image are used as explanatory variables and the past molten steel component is used as a response variable. The past inspection information and the past image are as described above. The prediction model may be generated by training an untrained model using these values. FIG. 10 is a diagram showing an example of the prediction model obtained by being trained using the training data as shown in FIG. 9. The current predicted molten steel component, which is a response variable, is obtained by inputting the current inspection information and the current image (for example, a scrap image before dissolution), which are explanatory variables, to the prediction model.

In addition, the inspection record information may include, for example, types and a number of foreign substances predicted to be mixed into the ferrous scrap, a grade of the scrap determined by a system that determines the grade, and composition data of all parts constituting the scrap. The composition data may, for example, be the proportions of part types such as sheet steel, reinforcing bar, and H-beam steel.

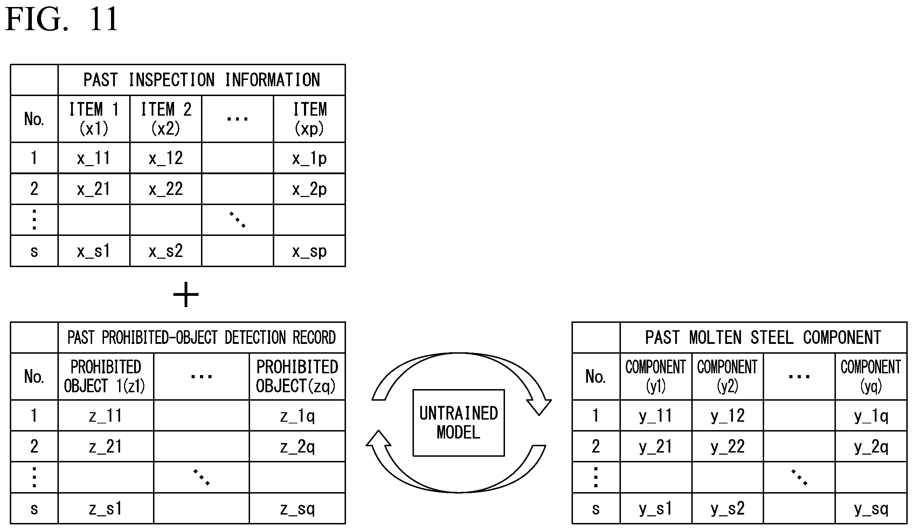

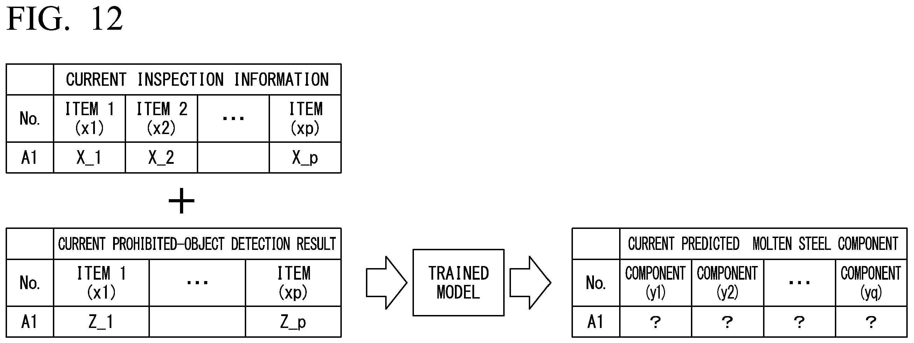

FIG. **11** is a diagram showing a fourth specific example of the training data. The training data shown in FIG. 11 is an example in which the past inspection information and a past prohibited-object detection record are used as explanatory variables and the past molten steel component is used as a response variable. The past inspection information is as described above. The past prohibited-object detection record includes values related to a plurality of prohibited objects (tramp element components z1 to zq) for each lot indicated as No. The past molten steel component has values of components (y1 to yq) actually molten and measured for each lot of the past inspection information. The prediction model may be generated by training an untrained model using these values. FIG. 12 is a diagram showing an example of the prediction model obtained by being trained using the training data as shown in FIG. 11. The current predicted molten steel component, which is a response variable, is obtained by inputting the current inspection information and a current prohibited-object detection result, which are explanatory variables, to the prediction model. The current inspection information indicates values of the items x1 to xp related to the ferrous scrap that is the prediction target. The current prohibited-object detection result indicates values of the items z1 to zq related to the prohibited object of the ferrous scrap that is the prediction target. The current predicted molten steel component indicates a predicted value of each component related to the ferrous scrap that is the prediction target.

The training data is not limited to the above-described examples, and various types of supplementary information may be used. Additionally, information indicating a generation cause of the ferrous scrap or information indicating the delivery contractor may be used as the training data. Examples of the generation cause include a cause that the ferrous scrap is generated by demolishing the building and a cause that the ferrous scrap is generated as waste during processing of steel.

The prohibited object of the ferrous scrap includes impurities such as so-called tramp elements, sealed substances (gas cylinders and the like) that are dangerous to explode during the dissolution, and iron with a large amount of non-conductors (insulating materials and the like) attached. Among these prohibited objects, a substance containing the tramp elements is relatively large. Therefore, the concentration of the impurity elements contained in the ferrous scrap is correlated with the amount of the prohibited objects contained in the ferrous scrap. Some of the prohibited objects contained in the ferrous scrap are removed by the sorting operator of the delivery contractor in a process until the ferrous scrap is delivered. Therefore, the amount of the prohibited objects contained in the ferrous scrap is correlated with the delivery contractor. For example, this is because, among a plurality of delivery contractors, there are a delivery contractor that removes a larger amount of the prohibited objects and a delivery contractor that removes a smaller amount of the prohibited objects. In addition, the amount of the prohibited objects contained in the ferrous scrap is also correlated with the sorting operator. This is because, even for the same delivery contractor, among the plurality of sorting operators belonging to that delivery contractor, some remove a larger amount of the prohibited objects while others remove a smaller amount of the prohibited objects.

The amount of the prohibited objects contained in the ferrous scrap greatly changes depending on what structure from which the ferrous scrap is obtained. Therefore, the amount of the prohibited objects contained in the ferrous scrap is correlated with the structure (generation source) from which the ferrous scrap is obtained. In addition, the ferrous scrap obtained from the same structure is concentratedly delivered at a time close to a time of demolition of the structure. Therefore, the amount of the prohibited objects contained in the ferrous scrap is correlated with a time when the ferrous scrap is delivered or a time when the ferrous scrap is generated.

The types and amount of the impurities contained in the ferrous scrap can be predicted based on information on a shape or a color, which is information obtained from the external appearance. For example, it is possible to determine a grade of heavy scrap based on the shape (particularly, a thickness). In addition, an amount of rust, a color of a copper wire with a coating, or the like can be determined based on the color. In addition, the prohibited objects such as the motor and the power switchboard can be determined based on the information obtained from the external appearance. Therefore, the concentration of the impurity elements contained in the ferrous scrap is correlated with the information (two-dimensional image or the like) obtained from the external appearance.

The external appearance information is information acquired by the imaging device as described above. In the following description, an example will be described in which the two-dimensional image obtained by receiving the visible light is used as the external appearance information.

In the generation of the prediction model, both the inspection record information and the two-dimensional image (external appearance information) in the supplementary information may be used, or only one of the inspection record information or the two-dimensional image may be used. In a case where only the inspection record information is used for the generation of the prediction model, only the inspection record information is used for the prediction processing using the prediction model. In a case where only the two-dimensional image is used for the generation of the prediction model, only the two-dimensional image is used for the prediction processing using the prediction model. In a case where the inspection record information and the two-dimensional image are used for the generation of the prediction model, it is desirable that the inspection record information and the two-dimensional image are used for the prediction processing using the prediction model, but only one of the inspection record information or the two-dimensional image may be used. In addition, the information used as the external appearance information may be any information as long as the information can indicate the external appearance of the ferrous scrap. For example, information indicating a three-dimensional shape acquired by using a three-dimensional shape measurement system such as LIDAR may be used as the external appearance information. In addition, image information of another aspect, such as a multispectral image or an infrared image, may be used as the external appearance information.

In a case where only the inspection record information is used for the generation of the prediction model, a regression model such as multiple regression or a decision tree may be used as the prediction model. Alternatively, a model that outputs a predicted value in a probability distribution, such as Gaussian process regression, may be used. In addition, a classification model such as a support vector machine (SVM) that outputs which class among a plurality of classes indicating ranges of the predicted value belongs to may be used as the prediction model. In a case where only the two-dimensional image is used for the generation of the prediction model, a neural network such as a convolutional neural network (CNN) may be used as the prediction model, or another learning algorithm may be used. Even in a case where only the two-dimensional image is used for the generation of the prediction model, a regression model that outputs a predicted value may be used, or a classification model that outputs which class among a plurality of classes indicating ranges of the predicted value belongs to may be used.

In a case where the inspection record information and the two-dimensional image are used for the generation of the prediction model, a multimodal learning model (for example, multimodal deep learning) may be used as the prediction model. Specific examples of image-centric multimodal integration techniques (meta-information input techniques) include early-fusion and late-fusion. In early-fusion, the inspection record and the image information are converted into vectors of the same dimension such that one prediction model can be used. As a result, an inference result can be obtained from one prediction model. In late-fusion, a TE component prediction model (Gaussian process regression or the like) using the inspection record and a TE component prediction model (CNN or the like) using the image information are used independently, and a final prediction result is obtained by weighting each prediction result. The multimodal integration technique is not limited to these specific examples, and other techniques may be applied. Even in a case where the inspection record information and the two-dimensional image are used for the generation of the prediction model, a regression model that outputs a predicted value may be used, or a classification model that outputs which class among a plurality of classes indicating ranges of the prediction value belongs to may be used.

Next, the control unit 33 will be described. The control unit 33 is configured using one or more hardware processors such as a CPU and one or more memories (main storage devices). The memory is configured using, for example, a storage device such as a RAM or a read only memory (ROM). The control unit 33 functions by causing the one or more hardware processors to perform various computations through execution of one or more programs stored in the memories. The control unit 33 functions as, for example, an information control unit 331, a prediction unit 332, and an output control unit 333. All or some of the functions of the control unit 33 may be implemented by using hardware such as an ASIC, a PLD, or an FPGA. The above-described program may be recorded on a computer-readable recording medium. The computer-readable recording medium is, for example, a portable medium such as a flexible disk, a magneto-optical disk, a ROM, a CD-ROM, or a semiconductor storage device (for example, an SSD), or a storage device such as a hard disk or a semiconductor storage device built in a computer system. The above-described program may be transmitted via a telecommunications line.

The information control unit 331 acquires information from another device such as the terminal device 10 and the imaging device 20. Specific examples of the acquired information include the supplementary information of the target scrap. Specifically, the information control unit 331 may acquire any one or both of the inspection record information and the two-dimensional image (external appearance information). The information control unit 331 records the acquired information in the storage device such as the memory. The information control unit 331 transmits the information to another device such as the terminal device 10. Specific examples of the transmitted information include information predicted by the prediction unit 332. The exchange of information between the information control unit 331 and another device may be performed by, for example, communication via the communication unit 31.

Next, the prediction unit 332 will be described. The prediction unit 332 predicts the concentration of the impurity elements contained in the target scrap based on the supplementary information acquired by the information control unit 331 and the prediction model stored in the prediction model storage unit 321. A specific example of such processing will be described. The prediction unit 332 acquires the supplementary information corresponding to the prediction model. In other words, the prediction unit 332 acquires information corresponding to the explanatory variable of the known data used for the acquisition of the prediction model. The prediction unit 332 predicts the concentration of the impurity elements contained in the target scrap by using information (information included in the supplementary information) corresponding to the acquired explanatory variable and the prediction model.

The output control unit 333 outputs information indicating the prediction result of the prediction unit 332. The output control unit 333 may output, for example, information indicating the prediction result to the terminal device 10. The output control unit 333 may output information indicating the prediction result by controlling an output device connected to the prediction device 30. For example, the output control unit 333 may display a character string or an image indicating the prediction result on an image display device connected to the prediction device 30. For example, the output control unit 333 may output a voice indicating the prediction result to the speaker connected to the prediction device 30.

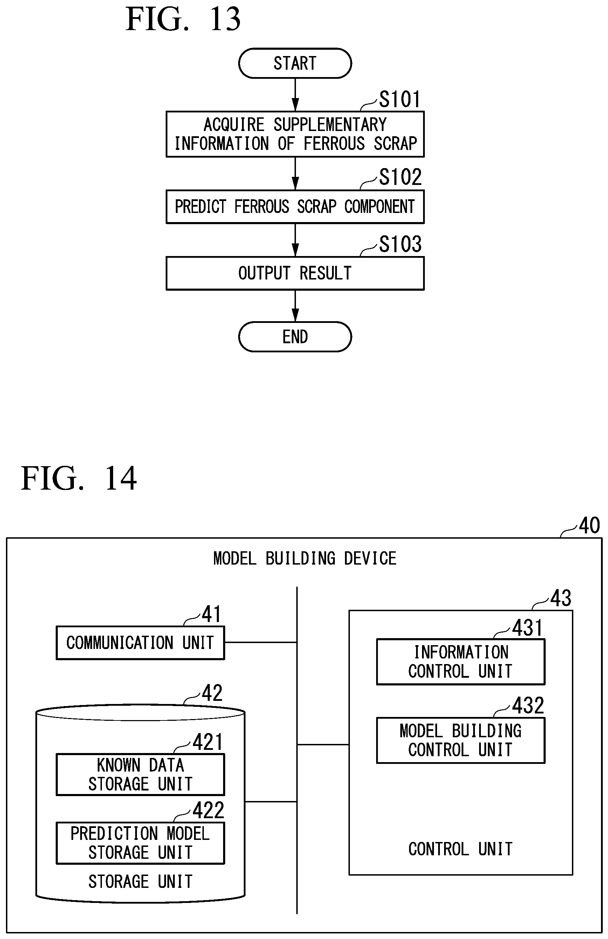

FIG. 13 is a flowchart showing a specific example of processing of the prediction device 30. First, the information control unit 331 acquires the supplementary information of the target scrap from another device (terminal device 10 or imaging device 20) (step S101). The prediction unit 332 predicts the concentration of the impurity elements contained in the target scrap by using the acquired supplementary information and the prediction model (step S102). The predicted concentration may be calculated by, for example, the following expression. Mass percent concentration (%) = (mass (g) of TE component/mass (g) of scrap). Then, the output control unit 333 outputs the prediction result (step S103).

FIG. 14 is a schematic block diagram showing a specific example of a functional configuration of the model building device 40. The model building device 40 is configured using, for example, an information processing device such as a personal computer or a server device. The model building device 40 includes a communication unit 41, a storage unit 42, and a control unit 43.

The communication unit 41 is a communication device. The communication unit 41 may be, for example, configured as a network interface. The communication unit 41 performs data communication with another device via the network 200 under the control of the control unit 43. The communication unit 41 may be a device that performs wireless communication or a device that performs wired communication.

The storage unit 42 is configured using a storage device such as a magnetic hard disk device or a semiconductor storage device. The storage unit 42 stores data used by the control unit 43. The storage unit 42 may function as, for example, a known data storage unit 421 and a prediction model storage unit 422.

The known data storage unit 421 stores known data used for the model building processing executed in the model building device 40. The known data may be, for example, the training data used in the supervised learning processing. The known data stored in the known data storage unit 421 is, for example, data including the supplementary information and the ground truth label related to the concentration of the impurity elements contained in the target scrap having the supplementary information. In this case, the supplementary information included in the known data corresponds to the explanatory variable, and the concentration of the impurity elements contained in the target scrap corresponds to the response variable. The supplementary information included in the known data may be, for example, the two-dimensional image (external appearance information) acquired by the imaging device 20.

The ground truth label included in the known data can be obtained by analyzing the ferrous scrap for which the supplementary information is obtained as the known data. Specifically, for example, the ground truth label can be obtained by the following analysis. For example, the amount of the impurities (for example, copper) contained in the ferrous scrap may be obtained by performing optical analysis such as laser, X-rays, and neutron rays on the entire ferrous scrap. For example, in a case of mixing and dissolving a plurality of ferrous scraps, the ground truth label of each known data may be calculated from the concentration measurement result of the impurity elements in the molten steel by using a plurality of blend-dissolution records in which the combination of the blend ratios of the mixed ferrous scraps is the same but only the blend ratio is changed. For example, the ground truth label may be obtained by dissolving a scrap lot for which the TE concentration is desired alone and measuring the component of the molten steel.

The prediction model storage unit 422 stores the prediction model obtained by the model building processing using the known data stored in the known data storage unit 421.

The control unit 43 is configured using a processor such as a CPU and a memory. The control unit 43 functions as an information control unit 431 and a model building control unit 432 by the processor executing a program. All or some of the functions of the control unit 43 may be implemented by using hardware such as an ASIC, a PLD, or an FPGA. The above-described program may be recorded on a computer-readable recording medium. The computer-readable recording medium is, for example, a portable medium such as a flexible disk, a magneto-optical disk, a ROM, a CD-ROM, or a semiconductor storage device (for example, an SSD), or a storage device such as a hard disk or a semiconductor storage device built in a computer system. The above-described program may be transmitted via a telecommunications line.

The information control unit 431 controls input and output of information. For example, the information control unit 431 acquires the known data from another device (information processing device or storage medium) and records the known data in the known data storage unit 421. For example, the information control unit 431 transmits the prediction model stored in the prediction model storage unit 422 to another device (for example, the prediction device 30).

The model building control unit 432 builds the prediction model by performing the model building processing using the known data stored in the known data storage unit 421. The model building control unit 432 may build the prediction model by executing the supervised learning processing using the supplementary information stored in the known data storage unit 421 as the training data. The processing executed by the model building control unit 432 is not limited to the supervised learning processing. For example, the model building control unit 432 may build the prediction model by performing the learning processing using a statistical method such as multivariate analysis using the known data.

As a specific example of such learning processing, for example, regression machine learning processing may be used, or other learning techniques such as a neural network or deep learning may be used. The model building control unit 432 records the generated prediction model in the prediction model storage unit 422. The prediction model obtained by the model building control unit 432 may be transmitted to the prediction device 30 and recorded in the prediction model storage unit 321 of the prediction device 30. Such a prediction model can obtain a predicted value of the concentration of the impurity elements corresponding to the prediction model as output by providing the explanatory variable including the supplementary information as input.

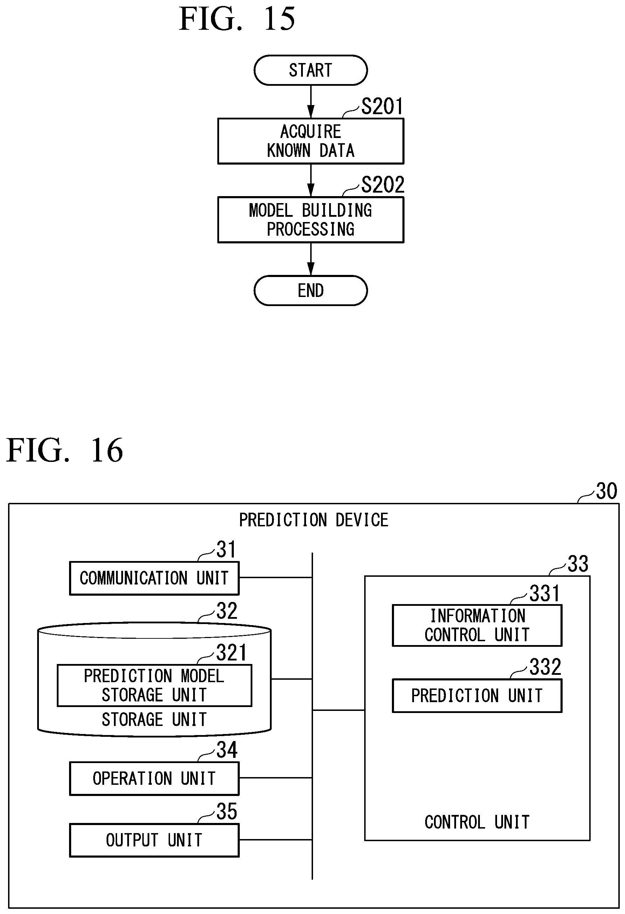

FIG. 15 is a flowchart showing a specific example of processing of the model building device 40. First, the information control unit 431 acquires the known data (step S201). The known data may be, for example, input by the user, acquired by communication from another information device, or acquired from a recording medium connected to the model building device 40. The model building control unit 432 executes the model building processing using the known data and records the prediction model in the prediction model storage unit 422 (step S202).

In the prediction system 100 configured as described above, the concentration of the impurity elements contained in the target scrap is predicted based on the prediction model obtained based on the known data of the other ferrous scrap and the supplementary information of the target scrap. Therefore, it is possible to predict the concentration with higher accuracy than a method of predicting the concentration of the impurity elements in the target scrap by using the image or the supplementary information alone.

In addition, in the prediction system 100, the concentration of the impurity elements contained in the target scrap is predicted based on information obtained without dissolving the target scrap. Therefore, in the prediction system 100, it is possible to predict the concentration of the impurity elements contained in the target scrap before the target scrap is actually dissolved. Therefore, it is possible to appropriately blend a plurality of target scraps based on the concentration of the impurity elements, and it is possible to prevent the concentration value of the impurity elements after the dissolution from being out of a standard value.

In addition, by performing the prediction before the target scrap is actually dissolved in the prediction system 100, it is possible to prevent the mixing of copper and the like in advance. In addition, since the prediction is possible even before the scrap is dissolved, in a case where the TE component at or above the standard is measured, it is possible to take measures such as adding a process of removing the sorting of the prohibited object causing the problem.

### (Modification Example)

In the present embodiment, the terminal device 10 and the prediction device 30 are configured as different devices, but may be configured as an integrated device. FIG. 16 is a diagram showing a modification example of the prediction device 30 configured as described above. The prediction device 30 shown in FIG. 16 includes an operation unit 34 and an output unit 35. The operation unit 34 and the output unit 35 of the prediction device 30 shown in FIG. 16 function in the same manner as the operation unit 12 and the output unit 13 of the terminal device 10, respectively. The control unit 33 operates in accordance with the operation of the operation unit 34, and outputs the information using the output unit 35.

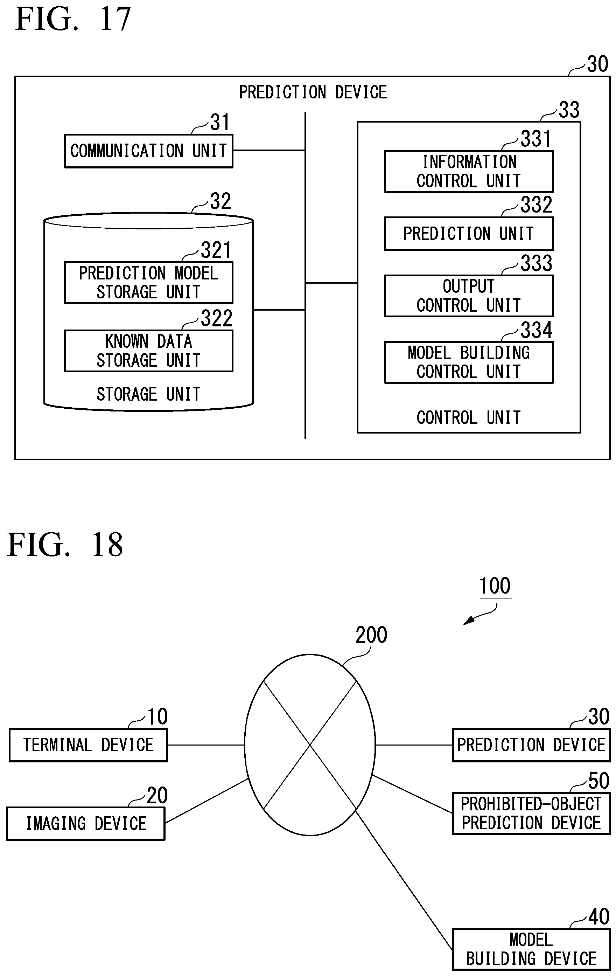

In the present embodiment, the prediction device 30 and the model building device 40 are configured as different devices, but may be configured as an integrated device. FIG. 17 is a diagram showing a modification example of the prediction device 30 configured as described above. The storage unit 32 of the prediction device 30 shown in FIG. 17 also functions as a known data storage unit 322. The control unit 33 of the prediction device 30 shown in FIG. 17 also functions as a model building control unit 334. The known data storage unit 322 functions in the same manner as the known data storage unit 421 of the model building device 40. The model building control unit 334 functions in the same manner as the model building control unit 432 of the model building device 40.

The prediction device 30 may be implemented by using a plurality of information processing devices. For example, the prediction device 30 may be implemented by using a device such as a cloud. For example, in the prediction device 30, the storage unit 32 and the control unit 33 may be implemented by different information processing devices. For example, the storage unit 32 of the prediction device 30 may be implemented by the plurality of information processing devices in a distributed manner. The model building device 40 may be implemented by using a plurality of information processing devices. For example, the model building device 40 may be implemented by using a device such as a cloud. For example, in the model building device 40, the storage unit 42 and the control unit 43 may be implemented by different information processing devices. For example, the storage unit 42 of the model building device 40 may be implemented by the plurality of information processing devices in a distributed manner.

The prediction model stored in the prediction model storage unit 321 of the prediction device 30 may be updated at a predetermined timing. For example, the prediction model storage unit 321 may be configured to store the prediction model generated based only on the known data obtained for the ferrous scrap generated within a predetermined period going back in time. With the configuration described above, it is possible to perform the prediction processing by using the prediction model in which the latest supplementary information is reflected. As a result, the accuracy of the prediction processing can be improved. As another specific example of the predetermined timing, there is a timing when it is detected that a change in the trend of the data has become significant enough to satisfy predetermined criteria. More specifically, for example, the predetermined timing may be a timing when the change satisfying the predetermined criteria is detected by performing unsupervised learning, or another algorithm for detecting a change in the trend may be used.

A spectral sensor may be used instead of the imaging device 20. In this case, spectral information of light received from the target scrap is obtained as the external appearance information. In this case, the prediction model obtained by performing the model building processing using the known data including the same spectral information is used for the prediction processing. The prediction device 30 performs the prediction processing using the spectral information obtained from the spectral sensor. The prediction device 30 may perform the prediction processing using the spectral information without using the supplementary information, or may perform the prediction processing using the supplementary information and the spectral information. The prediction device 30 may perform the prediction processing using the supplementary information, the spectral information, and the image data. In any case, the model building processing is performed using known information including each information to be used. With the configuration described above, it is possible to acquire information (for example, information related to a material of a member contained in the ferrous scrap) that is difficult to detect from the information obtained by the visible light or the information on the three-dimensional shape, and to execute the prediction processing with higher accuracy.

### [Second Embodiment]

FIG. 18 is a schematic block diagram showing a system configuration of the prediction system 100 according to a second embodiment of the present invention. The prediction system 100 according to the second embodiment further includes a prohibited-object prediction device 50. The prohibited-object prediction device 50 predicts information related to the prohibited objects contained in the target scrap. Specific examples of the information related to the prohibited object include the types of the prohibited objects contained in the target scrap, the number of the prohibited objects for each type contained in the target scrap, and the total number of the prohibited objects contained in the target scrap. The information related to the prohibited object may include all of the above-described information or may include a part of the above-described information. In the following description, as an example, an aspect will be described in which the types of the prohibited objects contained in the target scrap and the number of the prohibited objects for each type contained in the target scrap are included as the information related to the prohibited objects. The prediction device 30 further performs the prediction processing using the prediction result of the prohibited-object prediction device 50. The second embodiment of the prediction system 100 will be described below.

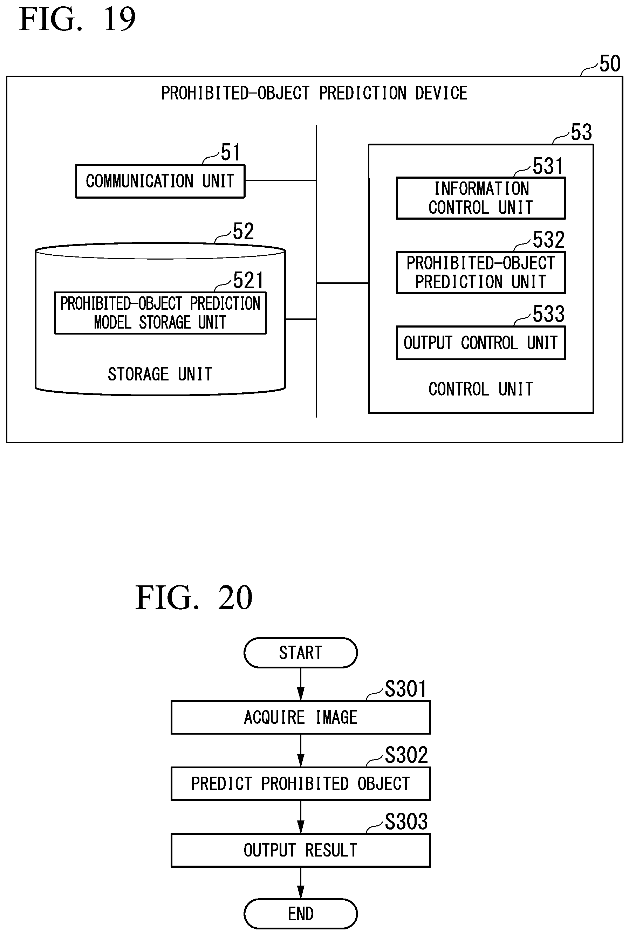

The prohibited-object prediction device 50 predicts the prohibited objects contained in the target scrap. The prohibited-object prediction device 50 is configured using, for example, an information processing device such as a personal computer or a server device. FIG. 19 is a schematic block diagram showing a specific example of a functional configuration of the prohibited-object prediction device 50. As in the shown example, the prohibited-object prediction device 50 includes a communication unit 51, a storage unit 52, and a control unit 53.

The communication unit 51 is a communication device. The communication unit 51 may be, for example, configured as a network interface. The communication unit 51 performs data communication with another device via the network 200 under the control of the control unit 53. The communication unit 51 may be a device that performs wireless communication or a device that performs wired communication.

The storage unit 52 is configured using a storage device such as a magnetic hard disk device or a semiconductor storage device. The storage unit 52 stores data used by the control unit 53. The storage unit 52 may function as, for example, a prohibited-object prediction model storage unit 521.

The prohibited-object prediction model storage unit 521 stores information (hereinafter, referred to as an "prohibited-object prediction model") used to predict the prohibited objects contained in the target scrap. The prohibited-object prediction model is generated in advance by the model building processing. Such model building processing may be executed, for example, by another device (for example, the model building device 40), by the device (prohibited-object prediction device 50) itself, or by human labor. The prohibited-object prediction model stored in the prohibited-object prediction model storage unit 521 will be described below.

The prohibited-object prediction model may be obtained by, for example, the model building processing using known data. The prohibited-object prediction model may be information indicating a trained model obtained by performing supervised learning processing using a plurality of known data (training data) having ground truth labels, information obtained by performing statistical processing using known data, or other types of information. Specific examples of the learning processing include multivariate analysis, so-called machine learning, and deep learning. As the learning processing, learning processing for classification may be used or learning processing for regression may be used in accordance with the ground truth label used in the training data. The control unit 53 predicts the prohibited objects contained in the target scrap by using the prediction model stored in the prohibited-object prediction model storage unit 521. The training data in the present embodiment will be described below.

As the training data, a combination of the images of the prohibited objects and the ground truth labels (information indicating the types of the prohibited objects contained in the image) is used. The prohibited object of the ferrous scrap is an object containing a large amount of impurities such as so-called tramp elements. As the image of the prohibited object, an image in which the prohibited object alone is captured may be used, or an image of the prohibited object mixed into the ferrous scrap may be used. In addition, the image of the prohibited object mixed into the ferrous scrap may be generated by superimposing the image of the prohibited object alone on the image of the ferrous scrap by the combination, and the generated image may be used as the image of the prohibited object. In these cases, a portion (partial image) of the image of the target scrap may be input to the prohibited-object prediction model, and a prediction result indicating whether or not the prohibited object is contained in the partial image may be output.

As the training data, a combination of the images of the target scrap and the ground truth labels (information indicating the types of the prohibited objects contained in the image) may be used. For example, an image of an aggregate of a plurality of ferrous scraps, such as an image captured by the imaging device 20, may be used. In this case, the ground truth label may include the number of the prohibited objects for each type contained in the target scrap shown in the image.

Next, the control unit 53 will be described. The control unit 53 is configured using one or more hardware processors such as a CPU and one or more memories (main storage devices). The memory is configured using, for example, a storage device such as a RAM or a read only memory (ROM). The control unit 53 functions by causing the one or more hardware processors to perform various computations through execution of one or more programs stored in the memories. The control unit 53 functions as, for example, an information control unit 531, a prohibited-object prediction unit 532, and an output control unit 533. All or some of the functions of the control unit 53 may be implemented by using hardware such as an ASIC, a PLD, or an FPGA. The above-described program may be recorded on a computer-readable recording medium. The computer-readable recording medium is, for example, a portable medium such as a flexible disk, a magneto-optical disk, a ROM, a CD-ROM, or a semiconductor storage device (for example, an SSD), or a storage device such as a hard disk or a semiconductor storage device built in a computer system. The above-described program may be transmitted via a telecommunications line.

The information control unit 531 acquires information from another device such as the terminal device 10 and the imaging device 20. Specific examples of the acquired information include an image captured by the imaging device 20. The information control unit 531 records the acquired information in the storage device such as the memory. The information control unit 531 transmits the information to another device such as the terminal device 10 and the prediction device 30. Specific examples of the transmitted information include information predicted by the prohibited-object prediction unit 532. The exchange of information between the information control unit 531 and another device may be performed by, for example, communication via the communication unit 51.

Next, the prohibited-object prediction unit 532 will be described. The prohibited-object prediction unit 532 predicts the prohibited objects contained in the target scrap based on the image acquired by the information control unit 531 and the prohibited-object prediction model stored in the prohibited-object prediction model storage unit 521. A specific example of such processing will be described. The prohibited-object prediction unit 532 acquires an image corresponding to the prohibited-object prediction model. In other words, the prohibited-object prediction unit 532 acquires information corresponding to the explanatory variable of the known data used for the acquisition of the prohibited-object prediction model. The prohibited-object prediction unit 532 predicts the prohibited objects contained in the target scrap by using information (image of the same size) corresponding to the acquired explanatory variable and the prohibited-object prediction model.

The output control unit 533 outputs information indicating the prediction result of the prohibited-object prediction unit 532. The output control unit 533 outputs information by transmitting the information indicating the prediction result to the prediction device 30. The output control unit 533 may further transmit the information indicating the prediction result to a device such as the terminal device 10. The output control unit 533 may output the information indicating the prediction result by controlling an output device connected to the prohibited-object prediction device 50. For example, the output control unit 533 may display a character string or an image indicating the prediction result on an image display device connected to the prohibited-object prediction device 50. For example, the output control unit 533 may output a voice indicating the prediction result to the speaker connected to the prohibited-object prediction device 50.

FIG. 20 is a flowchart showing a specific example of processing of the prohibited-object prediction device 50. First, the information control unit 531 acquires the image of the target scrap from another device (imaging device 20) (step S301). The prohibited-object prediction unit 532 predicts the prohibited objects contained in the target scrap by using the acquired image and the prohibited-object prediction model (step S302). Then, the output control unit 533 outputs the prediction result to the prediction device 30 (step S303).

Unlike the first embodiment, the prediction unit 332 of the prediction device 30 shown in FIG. 18 in the second embodiment uses the information related to the prohibited objects contained in the target scrap as one of the supplementary information. Specifically, the prediction unit 332 performs the prediction processing of the concentration of the impurity elements in the target scrap by using information indicating the types and the number of the prohibited objects contained in the target scrap as the explanatory variable. The prediction result of the prohibited-object prediction device 50 is used as the information used for the prediction processing. That is, the prediction unit 332 receives the prediction result from the prohibited-object prediction device 50, and the prediction model in the second embodiment is generated by performing the model building processing using the known data including the information related to the prohibited object in the supplementary information. Therefore, the model building device 40 in the second embodiment stores the known data including the information related to the prohibited object in the supplementary information in the known data storage unit 421, and the model building control unit 432 generates the prediction model using the known data.

In the prediction system 100 configured as described above, in a case of predicting the concentration of the impurity elements contained in the target scrap, information (for example, the types and the number) related to the prohibited object predicted to be contained in the target scrap is used as the explanatory variable. Since a large amount of impurities are contained in the prohibited object, it can be said that the information related to the prohibited objects contained in the target scrap is highly correlated with the concentration of the impurity elements contained in the target scrap. Therefore, with the configuration described above, it is possible to predict the concentration of the impurity elements in the target scrap with higher accuracy.

### (Modification Example)

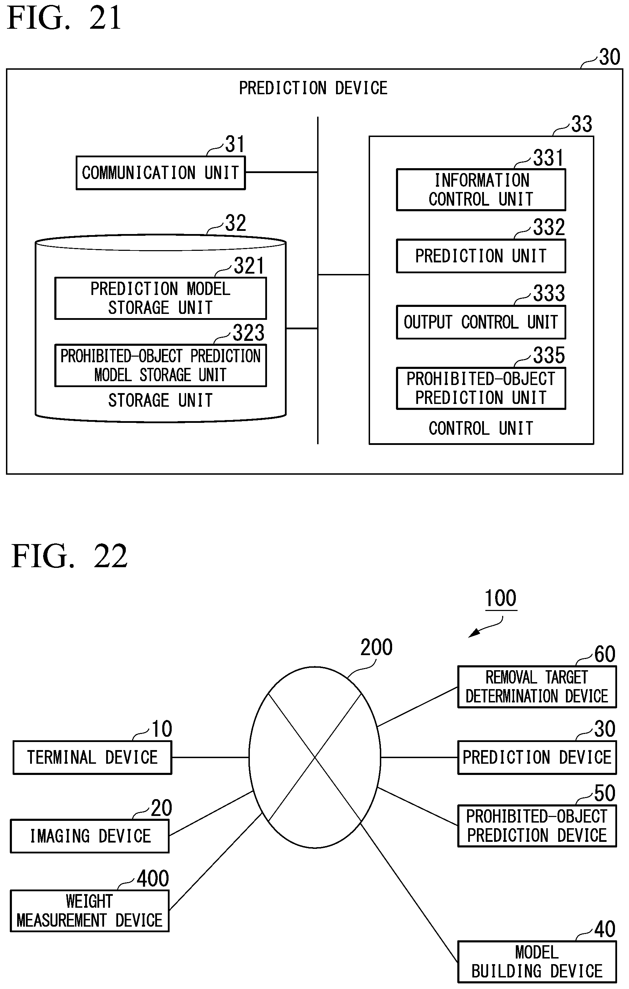

In the present embodiment, the prediction device 30 and the prohibited-object prediction device 50 are configured as different devices, but may be configured as an integrated device. FIG. 21 is a diagram showing a modification example of the prediction device 30 configured as described above. The storage unit 32 of the prediction device 30 shown in FIG. 21 also functions as a prohibited-object prediction model storage unit 323. The control unit 33 of the prediction device 30 shown in FIG. 21 also functions as a prohibited-object prediction unit 335. The prohibited-object prediction model storage unit 323 functions in the same manner as the prohibited-object prediction model storage unit 521 of the prohibited-object prediction device 50. The prohibited-object prediction unit 335 functions in the same manner as the prohibited-object prediction unit 532 of the prohibited-object prediction device 50.

The prohibited-object prediction device 50 may be implemented by using a plurality of information processing devices. For example, the prohibited-object prediction device 50 may be implemented by using a device such as a cloud. For example, in the prohibited-object prediction device 50, the storage unit 52 and the control unit 53 may be implemented by different information processing devices. For example, the storage unit 52 of the prohibited-object prediction device 50 may be implemented by the plurality of information processing devices in a distributed manner.

### [Third Embodiment]

FIG. 22 is a schematic block diagram showing a system configuration of the prediction system 100 according to a third embodiment of the present invention. The prediction system 100 according to the third embodiment further includes a removal target determination device 60 and a weight measurement device 400 with respect to the second embodiment of the prediction system 100. An upper limit value (hereinafter, referred to as an "upper limit impurity value") of the concentration of the impurity elements that can be allowed in the target scrap is set in the removal target determination device 60. The upper limit impurity value may be determined as the concentration or may be determined as an absolute amount (mass) per weight of the scrap to be handled. The removal target determination device 60 determines the prohibited object (hereinafter, referred to as a "removal target prohibited object") to be removed among the prohibited objects contained in the target scrap such that the concentration falls below the upper limit impurity value. By the worker removing the prohibited object from the target scrap in accordance with the determination result of the removal target determination device 60, it is possible to perform the adjustment such that the concentration of the impurity elements in the target scrap becomes equal to or less than the upper limit impurity value. The third embodiment of the prediction system 100 will be described below.

The weight measurement device 400 measures the weight of the target scrap in the prediction system 100. The weight measurement device 400 may measure, for example, the weight of the target scrap itself. In this case, for example, the weight of the target scrap itself may be measured by placing the target scrap on a weighing scale as described below. The weight measurement device 400 may measure the weight of a container on which the target scrap is loaded together with the weight of the target scrap. In this case, the weight measurement device 400 may also measure the weight of the container after all the target scrap is transported out. The weight of the target scrap may be calculated by subtracting the measurement result of the weight of the container after all the target scrap is transported out from the measurement result of the weight of the container and the target scrap. Such a calculation may be performed by the weight measurement device 400 or may be performed by a device such as the prediction device 30 or the removal target determination device 60. The weight measurement device 400 may measure the weight of a moving body (for example, a truck vehicle or the like) on which the target scrap is loaded together with the weight of the target scrap. In this case, the weight measurement device 400 may also measure the weight of the moving body after all the target scrap is transported out. The weight of the target scrap may be calculated by subtracting the measurement result of the weight of the moving body after all the target scrap is transported out from the measurement result of the weight of the moving body and the target scrap. Such a calculation may be performed by the weight measurement device 400 or may be performed by a device such as the prediction device 30 or the removal target determination device 60.

The weight measurement device 400 may be configured using, for example, the weighing scale that has a device for loading the target scrap on an upper portion and measures the weight of an object loaded on the device. The weight measurement device 400 may be configured using, for example, a vehicle weighing scale that is embedded in the ground and measures the weight of a vehicle positioned on an upper portion thereof. Other configurations may be adopted in the weight measurement device 400. The weight measurement device 400 transmits the information indicating the measurement result to the prediction device 30 or the removal target determination device 60.

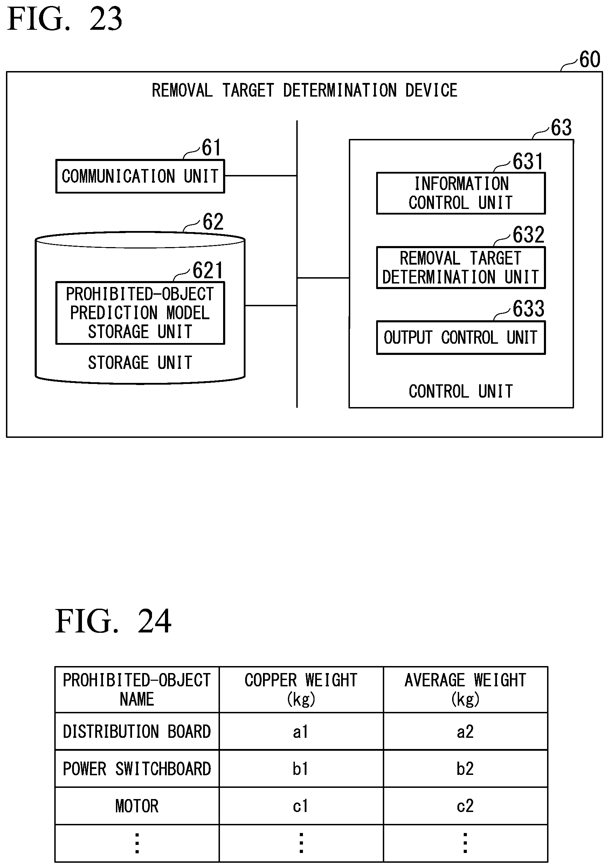

The removal target determination device 60 determines the removal target prohibited object among the prohibited objects contained in the target scrap such that the concentration falls below the upper limit impurity value. The removal target determination device 60 is configured using, for example, an information processing device such as a personal computer or a server device. FIG. 23 is a schematic block diagram showing a specific example of a functional configuration of the removal target determination device 60. As in the shown example, the removal target determination device 60 includes a communication unit 61, a storage unit 62, and a control unit 63.

The communication unit 61 is a communication device. The communication unit 61 may be, for example, configured as a network interface. The communication unit 61 performs data communication with another device via the network 200 under the control of the control unit 63. The communication unit 61 may be a device that performs wireless communication or a device that performs wired communication.

The storage unit 62 is configured using a storage device such as a magnetic hard disk device or a semiconductor storage device. The storage unit 62 stores data used by the control unit 63. The storage unit 62 may function as, for example, a prohibited-object information storage unit 621.

The prohibited-object information storage unit 621 stores prohibited-object information that is information related to the weight of the prohibited object. FIG. 24 is a diagram showing a specific example of the prohibited-object information. The prohibited-object information shown in FIG. 24 has three values of a prohibited-object name, a copper weight, and an average weight. The prohibited-object name indicates a name of the type of each prohibited object. The copper weight indicates an average value of the weights of the impurities (copper) contained in the various types of prohibited objects. In the present embodiment, copper is used as an example of the impurities contained in the prohibited object, but the weight of other impurities may be registered in the prohibited-object information. The average weight indicates an average value of the weights of the various types of prohibited objects. For example, it is known that, in the motor, about 20% of the total weight is copper. It is assumed that the same applies to other prohibited objects.

Next, the control unit 63 will be described. The control unit 63 is configured using one or more hardware processors such as a CPU and one or more memories (main storage devices). The memory is configured using, for example, a storage device such as a RAM or a read only memory (ROM). The control unit 63 functions by causing the one or more hardware processors to perform various computations through execution of one or more programs stored in the memories. The control unit 63 functions as, for example, an information control unit 631, a removal target determination unit 632, and an output control unit 633. All or some of the functions of the control unit 63 may be implemented by using hardware such as an ASIC, a PLD, or an FPGA. The above-described program may be recorded on a computer-readable recording medium. The computer-readable recording medium is, for example, a portable medium such as a flexible disk, a magneto-optical disk, a ROM, a CD-ROM, or a semiconductor storage device (for example, an SSD), or a storage device such as a hard disk or a semiconductor storage device built in a computer system. The above-described program may be transmitted via a telecommunications line.

The information control unit 631 acquires information from another device such as the terminal device 10, the prediction device 30, and the prohibited-object prediction device 50. Specific examples of the acquired information include the prediction result of the prohibited-object prediction device 50. As another specific example of the acquired information, there is information related to the upper limit impurity value. The information control unit 631 records the acquired information in the storage device such as the memory. The information control unit 631 transmits the information to another device such as the terminal device 10 and the prediction device 30. Specific examples of the transmitted information include information (information indicating the removal target prohibited object) determined by the removal target determination unit 632. The exchange of information between the information control unit 631 and another device may be performed by, for example, communication via the communication unit 61.

The removal target determination unit 632 determines the type and the amount of the removal target prohibited object from the target scrap based on the weight of the target scrap, the predicted value of the concentration of the impurity elements contained in the target scrap, the information related to the prohibited objects contained in the target scrap, and the upper limit impurity value. That is, the removal target determination unit 632 receives the predicted value of the concentration of the impurity elements contained in the target scrap, the information related to the prohibited objects contained in the target scrap, and the upper limit impurity value as input. The removal target determination unit 632 determines whether or not it is necessary to remove the prohibited object such that the concentration falls below the upper limit impurity value by performing predetermined processing (removal necessity determination processing), and outputs the determination result. For example, in a case where the processing is executed at a timing of unloading from the bed of the truck using the transport device 300, the processing may be executed such that the concentration of the impurity element falls below the upper limit impurity value for each lot lifted by the lifting magnet 301 for unloading.

In a case where the processing is executed at a timing of unloading the scrap from the bed of the truck using the transport device 300, the processing may be performed as follows. Each time the scrap is lifted from the bed of the truck by the lifting magnet 301 for unloading, the image of the target scrap loaded on the bed of the truck is acquired. Then, for each newly obtained image, the processing is executed such that the concentration of the impurity element falls below the upper limit impurity value. Each time the scrap is lifted by the lifting magnet 301, a new surface layer of the target scrap loaded on the bed of the truck is exposed and shown in the image. Therefore, it is possible to appropriately determine the target scrap that is loaded on the inside or the bottom and that is not obtained as the image at first when the truck arrives. An environment in which such a configuration is adopted is not limited to an environment in which the target scrap is lifted from the bed of the truck. For example, it can be applied to an environment in which a large amount of target scrap is loaded on a predetermined region such as the ground and the target scrap is transported to another region by the transport device such as the lifting magnet 301 from the predetermined region.

A specific example of the removal necessity determination processing will be described. First, the removal target determination unit 632 acquires the prediction result of the information related to the concentration of the impurity elements contained in the target scrap that is the determination target. Such a prediction result may be obtained, for example, by performing the prediction processing using the prediction model stored in the prediction model storage unit 321. The removal target determination unit 632 may determine whether or not the removal is necessary depending on whether or not the prediction result (predicted value) of the concentration of the impurity elements contained in the target scrap is less than a predetermined threshold value. For example, it is determined that the removal is not necessary in a case where the predicted value is less than the predetermined threshold value, and it is determined that the removal is necessary in a case where the predicted value is equal to or larger than the predetermined threshold value. The predetermined threshold value may be determined for each type of the impurity elements.

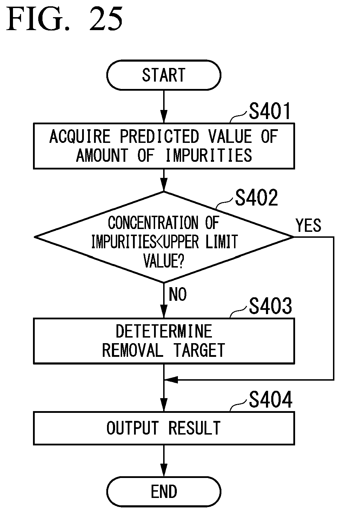

FIG. 25 is a flowchart showing a specific example of processing of the removal target determination unit 632. A specific example of the processing of the removal target determination unit 632 will be described below. The removal target determination unit 632 acquires the predicted value of the concentration of the impurity elements contained in the target scrap (step S401). The removal target determination unit 632 determines whether or not the predicted value of the concentration of the impurity elements is less than the upper limit impurity value (step S402). In a case where the predicted value of the concentration of the impurity elements exceeds the upper limit impurity value (step S402-NO), the removal target determination unit 632 determines the prohibited object (removal target prohibited object) to be removed in order to make the concentration of the impurity elements fall below the upper limit impurity value (step S403).

In the processing of step S403, for example, the removal target determination unit 632 determines the removal target prohibited object such that the concentration of the impurity elements in the target scrap after the removal target prohibited object is removed is less than the upper limit value. For example, by referring to the prohibited-object information stored in the prohibited-object information storage unit 621, the average value of the impurities (copper) contained in the various types of prohibited objects and the average value of the weights of the prohibited objects itself are known. In a case where the upper limit impurity value is given as a percentage, the total amount of the impurities to be removed can be calculated by multiplying the weight of the target scrap by a value obtained by subtracting the upper limit impurity value from the predicted value of the concentration of the impurity elements. The removal target determination unit 632 determines the removal target prohibited object such that the total amount of the impurities contained in the prohibited object that is actually removed exceeds the total amount calculated here.

In this case, in a case where the prediction result of the prohibited-object prediction device 50 is obtained, the removal target determination unit 632 may select the removal target prohibited object from the types and the number of the prohibited objects contained in the prediction result of the prohibited-object prediction device 50. By the removal target determination unit 632 determining the removal target prohibited object in this way, it is possible to output information related to the prohibited object that can be realistically removed to the worker, and it is easy to make the concentration of the impurity elements contained in the target scrap less than the upper limit impurity value.

The output control unit 633 outputs information indicating the determination result of the removal target determination unit 632. The output control unit 633 may output, for example, information indicating the determination result to the terminal device 10. The output control unit 633 may output information indicating the determination result by controlling an output device connected to the removal target determination device 60. For example, the output control unit 633 may display a character string or an image indicating the determination result on an image display device connected to the removal target determination device 60. For example, the output control unit 633 may output a voice indicating the determination result to the speaker connected to the removal target determination device 60.

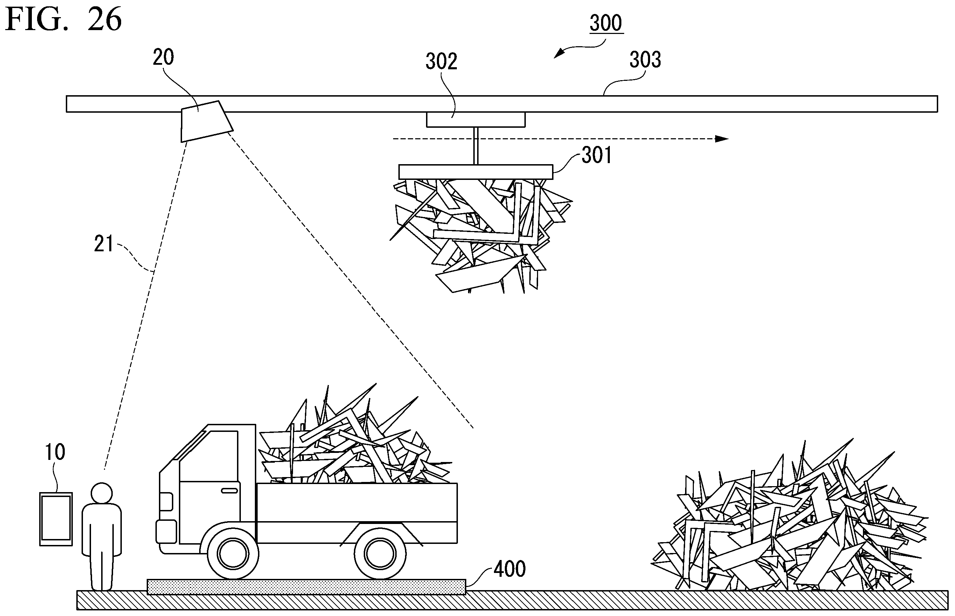

FIG. 26 is a diagram showing an application example of the prediction system 100 according to the present embodiment. In the application example shown in FIG. 26, the weight measurement device 400 is further provided with respect to the application example shown in FIG. 2. In FIG. 26, for example, the weight measurement device 400 is provided within an imaging range of the imaging device 20. By providing the weight measurement device 400 in this way, it is possible to image the target scrap while the weight is being measured in the weight measurement device 400. The determination result of the removal target determination device 60 may be transmitted to the terminal device 10. In this case, a person (worker) who operates the terminal device 10 may remove the removal target prohibited object from the target scrap based on the information indicating the removal target prohibited object output in the terminal device 10.

In the prediction system 100 configured as described above, the removal target determination device 60 outputs a specific example of the type and the amount of the prohibited object to be removed such that the amount contained in the target scrap is less than the upper limit impurity value. Therefore, it is easy to remove the prohibited object such that the requirement of the upper limit impurity value is satisfied.

### [Fourth embodiment]

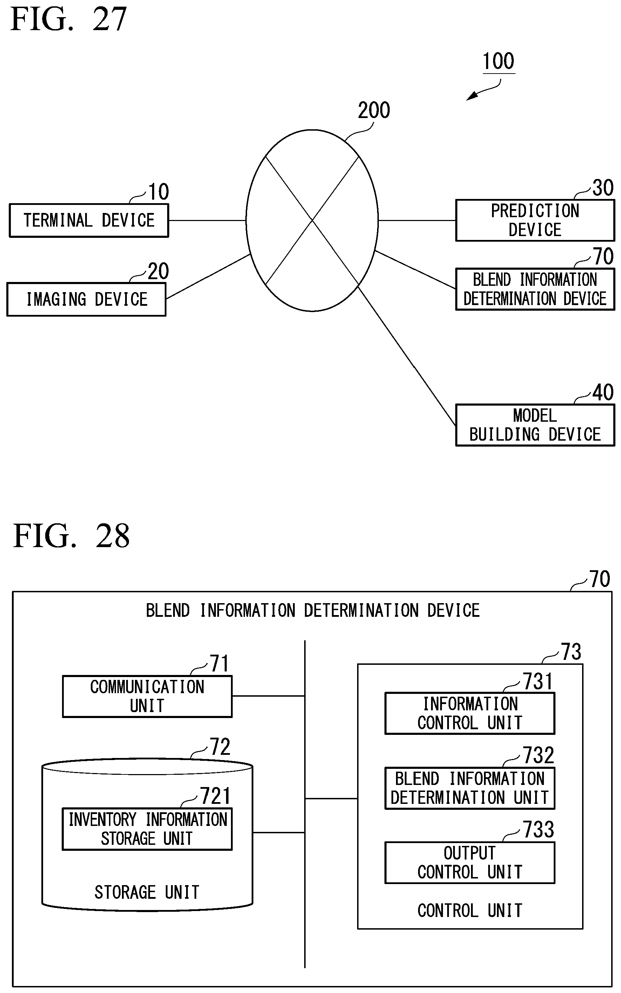

FIG. 27 is a schematic block diagram showing a system configuration of the prediction system 100 according to a fourth embodiment of the present invention. The prediction system 100 according to the fourth embodiment further includes a blend information determination device 70 with respect to the first embodiment of the prediction system 100. The blend information determination device 70 determines a blend ratio of the scrap of a plurality of lots for which the concentration of the impurity elements is predicted as the target scrap to satisfy predetermined criteria. By evaluating the blend with reference to the output of the blend information determination device 70, a person who determines the blend of the scrap can more easily and more accurately perform steelmaking using the scrap. The fourth embodiment of the prediction system 100 will be described below.

The blend information determination device 70 determines the blend of the scrap of the plurality of lots. The blend information determination device 70 is configured using, for example, an information processing device such as a personal computer or a server device. FIG. 28 is a schematic block diagram showing a specific example of a functional configuration of the blend information determination device 70. As in the shown example, the blend information determination device 70 includes a communication unit 71, a storage unit 72, and a control unit 73.

The communication unit 71 is a communication device. The communication unit 71 may be, for example, configured as a network interface. The communication unit 71 performs data communication with another device via the network 200 under the control of the control unit 73. The communication unit 71 may be a device that performs wireless communication or a device that performs wired communication.

The storage unit 72 is configured using a storage device such as a magnetic hard disk device or a semiconductor storage device. The storage unit 72 stores data used by the control unit 73. The storage unit 72 may function as, for example, an inventory information storage unit 721.

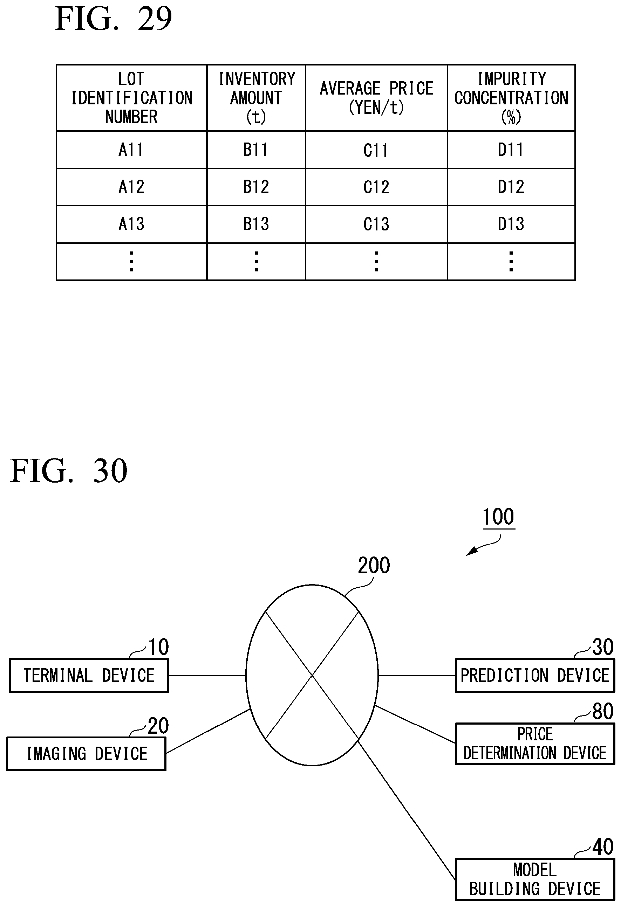

The inventory information storage unit 721 stores inventory information that is information related to the inventory of the ferrous scrap. FIG. 29 is a diagram showing a specific example of the inventory information. The inventory information shown in FIG. 29 includes four values of lot identification information, an inventory amount, an average unit price, and an impurity concentration. The lot identification information is identification information of each lot. The inventory amount indicates the inventory amount of the ferrous scrap belonging to each lot. The average unit price indicates the average unit price of the ferrous scrap belonging to each lot. For example, in a case where only one target scrap is contained in each lot, the price set for the target scrap is reflected in the average unit price. For example, in a case where a plurality of target scraps are contained in each lot, the average unit price is calculated based on the weight of the target scrap contained in each lot and the price at the time of purchase. The impurity concentration indicates an average value of the concentrations of the impurity elements contained in the ferrous scrap belonging to each lot. For example, in a case where only one target scrap is contained in each lot, the concentration of the impurity elements predicted in the target scrap is reflected in the impurity concentration. For example, in a case where a plurality of target scraps are contained in each lot, the impurity concentration is calculated based on the weight of the target scrap contained in each lot and the predicted value of the concentration of the impurity elements.

Next, the control unit 73 will be described. The control unit 73 is configured using one or more hardware processors such as a CPU and one or more memories (main storage devices). The memory is configured using, for example, a storage device such as a RAM or a read only memory (ROM). The control unit 73 functions by causing the one or more hardware processors to perform various computations through execution of one or more programs stored in the memories. The control unit 73 functions as, for example, an information control unit 731, a blend information determination unit 732, and an output control unit 733. All or some of the functions of the control unit 73 may be implemented by using hardware such as an ASIC, a PLD, or an FPGA. The above-described program may be recorded on a computer-readable recording medium. The computer-readable recording medium is, for example, a portable medium such as a flexible disk, a magneto-optical disk, a ROM, a CD-ROM, or a semiconductor storage device (for example, an SSD), or a storage device such as a hard disk or a semiconductor storage device built in a computer system. The above-described program may be transmitted via a telecommunications line.

The information control unit 731 acquires information from the other devices such as the terminal device 10 and the prediction device 30. Specific examples of the acquired information include information indicating a predetermined condition that should be satisfied in a case where molten steel is prepared for a desired kind of steel. The predetermined condition may indicate, for example, an upper limit value of impurities (for example, copper) that can be contained in the molten steel. The predetermined condition may include a condition related to the price. For example, the predetermined condition may include an upper limit value of the price, or a condition of "as low as possible" for the price. The price in the present embodiment is a price that has already been determined from the market conditions, the purchase history, and the like, and is different from a price (buying price) used in a sale and purchase to be performed between the vendors. The information control unit 731 records the acquired information in the storage device such as the memory. The information control unit 731 transmits the information to another device such as the terminal device 10 and the prediction device 30. Specific examples of the transmitted information include a determination result of the blend information. The exchange of information between the information control unit 731 and another device may be performed by, for example, communication via the communication unit 71.

The blend information determination unit 732 determines the ferrous scrap blend such that the designated predetermined condition is satisfied. For example, the blend information determination unit 732 may determine the blend using a predetermined algorithm (for example, linear programming) under at least any one of the following predetermined conditions (constraint conditions). Specific examples of the predetermined condition include the following conditions.
· Condition related to concentration of impurity element contained in molten steel (for example, upper limit value)
· Condition related to total cost in total amount of ferrous scrap contained in molten steel (for example, minimization)
· Condition related to use amount of ferrous scrap with small inventory amount (for example, minimization)
· Use less amount of ferrous scrap with higher price at time of purchase
· Use more amount of ferrous scrap with lower price at time of purchase

For example, the blend may be determined as follows by using the inventory information shown in FIG. 29 with a target value of the total weight of the ferrous scrap used for the blend and an upper limit value of the concentration of the impurities (for example, copper) as the constraint conditions. First, the constraint conditions are obtained. As a premise, a case is assumed in which the new scrap is used as a specific example of the high-grade scrap (high-grade scrap) blended with the low-grade scrap (low-grade scrap). The high-grade scrap is not limited to the new scrap, and for example, molten iron obtained from a blast furnace may be used, or other materials may be used. In this case, the average impurity concentration (D high-grade scrap) and the average unit price of the high-grade scrap (new scrap) are obtained, and the inventory amount of the high-grade scrap is unlimited. In this case, the following problem is solved using the impurity concentrations (for example, D11, D12, and D13) of each lot and the use amounts (for example, t1, t2, and t3) of each lot. Here, "C high-grade scrap" indicates the average unit price of the high-grade scrap, "D high-grade scrap" indicates the impurity concentration of the high-grade scrap, "t high-grade scrap" indicates the weight of the high-grade scrap to be used, and "tn" indicates the weight of each lot n to be used. $\begin{matrix}Σ \left(Dn\times tn\right) + D high - grade scrap \times t high - grade scrap \\ = \left(\begin{matrix}D 11 \times t 1 + D 12 \times t 2 + D 13 \times t 3 + D high - grade scrap \times t high - grade \\ scrap\end{matrix}\right) / \left(weight Σtn+t high-grade scrap\right) \leq upper limit value of impurity concentration\end{matrix}$

The constraint conditions are as follows.
1. Weight Σtn + t high-grade scrap satisfies target weight
2. Usage price Σ(average unit price n × tn) + C high-grade scrap × t high-grade scrap is minimized

In the above expression, "≤" indicates that the left side is equal to or less than the value of the right side.

All materials contained in the blend determined by the blend information determination unit 732 are not necessarily ferrous scrap. In addition, the inventory amount may be obtained based on delivery data (data indicating what is purchased and in what amount (for example, tonnage)). In addition, in a case where a material is used in which the exact amount of the impurities contained is known, the concentration of the impurity elements may be subtracted from the allowable amount of the impurities in the entire amount, and the ferrous scrap blend may be determined for the obtained difference.

The output control unit 733 outputs information indicating the determination result of the blend information determination unit 732. The output control unit 733 may output, for example, information indicating the determination result to the terminal device 10. The output control unit 733 may output information indicating the determination result by controlling an output device connected to the blend information determination device 70. For example, the output control unit 733 may display a character string or an image indicating the determination result on an image display device connected to the blend information determination device 70. For example, the output control unit 733 may output a voice indicating the determination result to the speaker connected to the blend information determination device 70.

In the prediction system 100 configured as described above, the blend information determination device 70 can easily determine appropriate blend for producing the molten steel satisfying a predetermined condition by using the inventory of the ferrous scrap. Therefore, it is possible to promote a demand for the ferrous scrap.

### [Fifth Embodiment]

FIG. 30 is a schematic block diagram showing a system configuration of the prediction system 100 according to a fifth embodiment of the present invention. The prediction system 100 according to the fifth embodiment further includes a price determination device 80 with respect to the first embodiment of the prediction system 100. The price determination device 80 determines the price of the target scrap based on the prediction result of the prediction device 30. The fifth embodiment of the prediction system 100 will be described below. The price determined by the price determination device 80 is, for example, a price (buying price) of a sale and purchase performed between a certain vendor and another vendor in a case where the vendor requests the other vendor to buy the ferrous scrap.

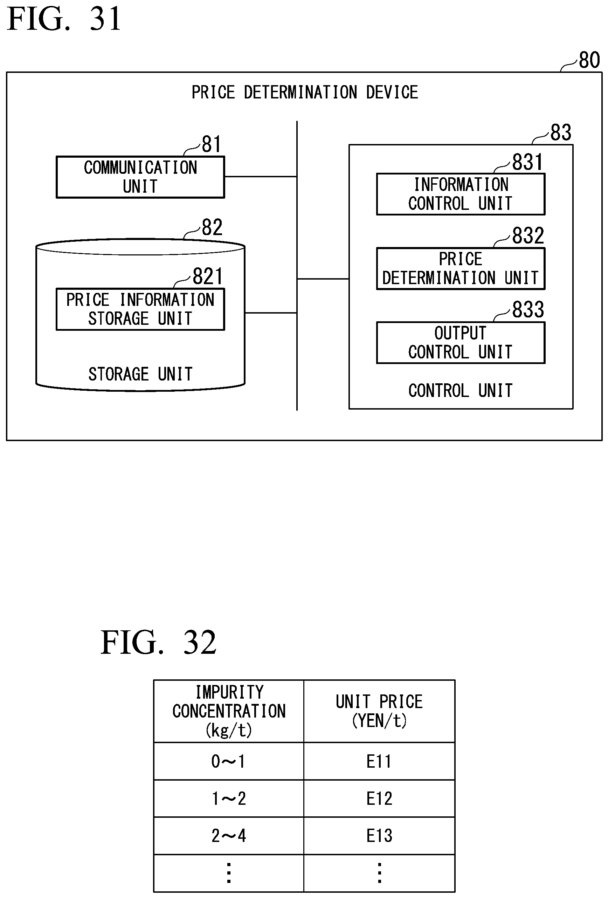

The price determination device 80 is configured using, for example, an information processing device such as a personal computer or a server device. FIG. 31 is a schematic block diagram showing a specific example of a functional configuration of the price determination device 80. As in the shown example, the price determination device 80 includes a communication unit 81, a storage unit 82, and a control unit 83.

The communication unit 81 is a communication device. The communication unit 81 may be, for example, configured as a network interface. The communication unit 81 performs data communication with another device via the network 200 under the control of the control unit 83. The communication unit 81 may be a device that performs wireless communication or a device that performs wired communication.

The storage unit 82 is configured using a storage device such as a magnetic hard disk device or a semiconductor storage device. The storage unit 82 stores data used by the control unit 83. The storage unit 82 may function as, for example, a price information storage unit 821.

The price information storage unit 821 stores price information that is information related to the price of the ferrous scrap. FIG. 32 is a diagram showing a specific example of the price information. The price information shown in FIG. 32 includes two values of the impurity concentration and the unit price. The impurity concentration indicates a range of the concentration of the impurity elements per unit weight (for example, 1 t) of the target scrap. The unit price indicates a unit price given to the target scrap in which the impurity concentration is within the range. By referring to the price information, the unit price can be obtained for each concentration of the impurity elements. That is, by determining the price using the price information, it is possible to determine the price corresponding to the impurity concentration in the target scrap.

Next, the control unit 83 will be described. The control unit 83 is configured using one or more hardware processors such as a CPU and one or more memories (main storage devices). The memory is configured using, for example, a storage device such as a RAM or a read only memory (ROM). The control unit 83 functions by causing the one or more hardware processors to perform various computations through execution of one or more programs stored in the memories. The control unit 83 functions as, for example, an information control unit 831, a price determination unit 832, and an output control unit 833. All or some of the functions of the control unit 83 may be implemented by using hardware such as an ASIC, a PLD, or an FPGA. The above-described program may be recorded on a computer-readable recording medium. The computer-readable recording medium is, for example, a portable medium such as a flexible disk, a magneto-optical disk, a ROM, a CD-ROM, or a semiconductor storage device (for example, an SSD), or a storage device such as a hard disk or a semiconductor storage device built in a computer system. The above-described program may be transmitted via a telecommunications line.

The information control unit 831 acquires information from the other devices such as the terminal device 10 and the prediction device 30. Specific examples of the acquired information include the prediction result of the concentration of the impurity elements by the prediction device 30. The information control unit 831 transmits the information to another device such as the terminal device 10 and the prediction device 30. Specific examples of the transmitted information include a determination result of the price of the target scrap. The exchange of information between the information control unit 831 and another device may be performed by, for example, communication via the communication unit 81.

The price determination unit 832 determines the unit price for the target scrap based on the prediction result of the prediction device 30. For example, the price determination unit 832 receives the prediction result of the concentration of the impurity elements by the prediction device 30 as input. The price determination unit 832 acquires the unit price corresponding to the impurity concentration indicated by the input prediction result based on the price information as shown in FIG. 32. In a case where the price determination unit 832 acquires information related to the weight of the target scrap from the weight measurement device 400, the price determination unit 832 may determine the price of the target scrap by multiplying the determined unit price by the weight.

The output control unit 833 outputs information indicating the determination result of the price determination unit 832. The output control unit 833 may output, for example, information indicating the determination result to the terminal device 10. The output control unit 833 may output information indicating the determination result by controlling an output device connected to the price determination device 80. For example, the output control unit 833 may display a character string or an image indicating the determination result on an image display device connected to the price determination device 80. For example, the output control unit 833 may output a voice indicating the determination result to the speaker connected to the price determination device 80.

In the prediction system 100 configured as described above, the price determination device 80 can determine the price corresponding to the impurity concentration in the target scrap.

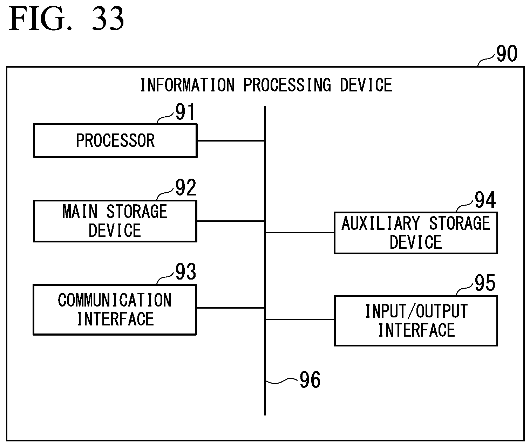

FIG. 33 is a diagram showing a schematic example of a hardware configuration of an information processing device 90 applied to the present embodiment. The information processing device 90 includes a processor 91, a main storage device 92, a communication interface 93, an auxiliary storage device 94, an input/output interface 95, and an internal bus 96. The processor 91, the main storage device 92, the communication interface 93, the auxiliary storage device 94, and the input/output interface 95 are communicably connected to each other via the internal bus 96. The information processing device 90 may be applied to, for example, the terminal device 10, the prediction device 30, the model building device 40, the prohibited-object prediction device 50, the removal target determination device 60, the blend information determination device 70, and the price determination device 80. In this case, for example, the communication unit of each device may be configured using the communication interface 93. For example, the storage unit of each device may be configured using the auxiliary storage device 94. In addition, the control unit of each device may be configured using the processor 91 and the main storage device 92.

Although the embodiments of the present invention have been described in detail with reference to the accompanying drawings, the specific configurations are not limited to the embodiments, and designs and the like within the scope of the present invention are also included.

### REFERENCE SIGNS LIST

100 Prediction system, 10 Terminal device, 20 Imaging device, 30 Prediction device, 31 Communication unit, 32 Storage unit, 321 Prediction model storage unit, 33 Control unit, 331 Information control unit, 332 Prediction unit, 333 Output control unit, 40 Model building device, 41 Communication unit, 42 Storage unit, 421 Known data storage unit, 422 Prediction model storage unit, 43 Control unit, 431 Information control unit, 432 Model building control unit, 50 Prohibited-object prediction device, 60 Removal target determination device, 70 Blend information determination device, 80 Price determination device

## Claims

1. A prediction device comprising a control unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap.

2. The prediction device according to Claim 1, wherein the supplementary information includes attribute information of the ferrous scrap itself or information related to an inspection performed in advance on the ferrous scrap.

3. The prediction device according to Claim 1 or 2, wherein the supplementary information includes information related to an external appearance of the ferrous scrap.

4. The prediction device according to Claim 1, wherein the supplementary information includes information related to prohibited objects contained in the ferrous scrap.

5. The prediction device according to Claim 4, wherein the information related to the prohibited objects contained in the ferrous scrap indicates number or types of the prohibited objects predicted by using a prohibited-object prediction model that is built in advance and is used to predict the prohibited objects.

6. A prediction method comprising a prediction step of predicting a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap.

7. A computer program for causing a computer to function as a prediction device including a control unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap.

8. A prediction system comprising:
a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and
a removal target determination unit configured to determine a prohibited object to be removed from the ferrous scrap based on a prediction result obtained by the prediction unit.

9. The prediction system according to Claim 8, wherein the supplementary information includes information related to an external appearance of the ferrous scrap.

10. A determination method comprising:
a prediction step of predicting a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and
a removal target determination step of determining a prohibited object to be removed from the ferrous scrap based on a prediction result obtained in the prediction step.

11. A computer program for causing a computer to function as a prediction system including:
a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and
a removal target determination unit configured to determine a prohibited object to be removed from the ferrous scrap based on a prediction result obtained by the prediction unit.

12. A prediction system comprising:
a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and
a blend information determination unit configured to determine a ferrous scrap blend such that a predetermined condition is satisfied, based on the prediction result obtained by the prediction unit and a price of the ferrous scrap.

13. A determination method comprising:
a prediction step of predicting a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and
a blend information determination step of determining a ferrous scrap blend such that a predetermined condition related to the ferrous scrap is satisfied, based on the prediction result obtained in the prediction step and the predetermined condition.

14. A computer program for causing a computer to function as a prediction system including:
a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and
a blend information determination unit configured to determine a ferrous scrap blend such that a predetermined condition is satisfied, based on the prediction result obtained by the prediction unit and a price of the ferrous scrap.

15. A prediction system comprising:
a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and
a price determination unit configured to determine a price of the ferrous scrap in accordance with a prediction result obtained by the prediction unit.

16. A determination method comprising:
a prediction step of predicting a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and
a price determination step of determining a price of the ferrous scrap in accordance with a prediction result obtained in the prediction step.

17. A computer program for causing a computer to function as a prediction system including:
a prediction unit configured to predict a concentration of impurity elements contained in ferrous scrap that is a determination target by using a prediction model built in advance using known data including supplementary information that is information related to ferrous scrap containing iron that is a recycling target and information related to a concentration of impurity elements contained in the ferrous scrap; and
a price determination unit configured to determine a price of the ferrous scrap in accordance with a prediction result obtained by the prediction unit.
